# EUROPEAN PATENT SPECIFICATION

(11) **EP 3 077 007 B1**
(45) Date of publication and mention of the grant of the patent: **20.11.2019**
(21) Application number: 14806237.5
(22) Date of filing: 01.12.2014
(51) Int. Cl.: A61K 47/60, A61K 9/127, A61P 35/00

(54) **REVERSIBLE PEGYLATION OF NANOCARRIERS**
REVERSIBLE PEGYLIERUNG VON NANOTRÄGERN
NANOSUPPORTS DE PEGYLATION RÉVERSIBLE

(30) Priority: 02.12.2013 EP 13195337
(43) Date of publication of application: 12.10.2016
(73) Proprietor: Albert-Ludwigs-Universität Freiburg, 79098 Freiburg (DE)
(72) Inventor: WEBER, Wilfried, 79100 Freiburg (DE); REBMANN, Balder, 79100 Freiburg (DE); ZURBRIGGEN, Matias, 79104 Freiburg (DE)
(74) Representative: Grindl, Wolfgang
(86) International application number: PCT/EP2014/076094
(87) International publication number: WO 2015/082396

(56) References cited:
- EP-A1- 2 455 104
- WO-A1-2009/108822
- HIROTO HATAKEYAMA ET AL: "A multifunctional envelope type nano device (MEND) for gene delivery to tumours based on the EPR effect: A strategy for overcoming the PEG dilemma", ADVANCED DRUG DELIVERY REVIEWS, ELSEVIER, AMSTERDAM, NL, vol. 63, no. 3, 3 September 2010 (2010-09-03), pages 152-160, XP028158142, ISSN: 0169-409X, DOI: 10.1016/J.ADDR.2010.09.001 [retrieved on 2010-09-15]
- SCHNYDER ANITA ET AL: "Targeting of skeletal muscle in vitro using biotinylated immunoliposomes", BIOCHEMICAL JOURNAL, PUBLISHED BY PORTLAND PRESS ON BEHALF OF THE BIOCHEMICAL SOCIETY, vol. 377, no. 1, 1 January 2004 (2004-01-01), pages 61-67, XP002560984, ISSN: 0264-6021, DOI: 10.1042/BJ20031034 [retrieved on 2003-09-30]
- HIROTO HATAKEYAMA ET AL: "The Polyethyleneglycol Dilemma: Advantage and Disadvantage of PEGylation of Liposomes for Systemic Genes and Nucleic Acids Delivery to Tumors", BIOLOGICAL & PHARMACEUTICAL BULLETIN, vol. 36, no. 6, 1 January 2013 (2013-01-01), pages 892-899, XP055114570, ISSN: 0918-6158, DOI: 10.1248/bpb.b13-00059

### SEQUENCE LISTING

<110> Albert-Ludwigs-Universitaet Freiburg
<120> Reversible PEGylation of nanocarriers
<130> P44771WO
<160> 19
<170> PatentIn version 3.5
<210> 1
   <211> 232
   <212> PRT
   <213> Artificial Sequence
<220>
   <223> Fc-domain
<400> 1
   Pro Arg Gly Pro Thr Ile Lys Pro Cys Pro Pro Cys Lys Cys Pro Ala
   1               5                   10                  15
   Pro Asn Leu Glu Gly Gly Pro Ser Val Phe Ile Phe Pro Pro Lys Ile
               20                  25                  30
   Lys Asp Val Leu Met Ile Ser Leu Ser Pro Ile Val Thr Cys Val Val
           35                  40                  45
   Val Asp Val Ser Glu Asp Asp Pro Asp Val Gln Ile Ser Trp Phe Val
       50                  55                  60
   Asn Asn Val Glu Val His Thr Ala Gln Thr Gln Thr His Arg Glu Asp
   65                  70                  75                  80
   Tyr Asn Ser Thr Leu Arg Val Val Ser Ala Leu Pro Ile Gln His Gln
                   85                  90                  95
   Asp Trp Met Ser Gly Lys Ala Phe Ala Cys Ala Val Asn Asn Lys Asp
               100                 105                 110
   Leu Pro Ala Pro Ile Glu Arg Thr Ile Ser Lys Pro Lys Gly Ser Val
           115                 120                 125
   Arg Ala Pro Gln Val Tyr Val Leu Pro Pro Pro Glu Glu Glu Met Thr
       130                 135                 140
   Lys Lys Gln Val Thr Leu Thr Cys Met Val Thr Asp Phe Met Pro Glu
   145                 150                 155                 160
   Asp Ile Tyr Val Glu Trp Thr Asn Asn Gly Lys Thr Glu Leu Asn Tyr
                   165                 170                 175
   Lys Asn Thr Glu Pro Val Leu Asp Ser Asp Gly Ser Tyr Phe Met Tyr
               180                 185                 190
   Ser Lys Leu Arg Val Glu Lys Lys Asn Trp Val Glu Arg Asn Ser Tyr
           195                 200                 205
   Ser Cys Ser Val Val His Glu Gly Leu His Asn His His Thr Thr Lys
       210                 215                 220
   Ser Phe Ser Arg Thr Pro Gly Lys
   225                 230
<210> 2
   <211> 220
   <212> PRT
   <213> GyrB
<400> 2
   Met Ser Asn Ser Tyr Asp Ser Ser Ser Ile Lys Val Leu Lys Gly Leu
   1               5                   10                  15
   Asp Ala Val Arg Lys Arg Pro Gly Met Tyr Ile Gly Asp Thr Asp Asp
               20                  25                  30
   Gly Thr Gly Leu His His Met Val Phe Glu Val Val Asp Asn Ala Ile
           35                  40                  45
   Asp Glu Ala Leu Ala Gly His Cys Lys Glu Ile Ile Val Thr Ile His
       50                  55                  60
   Ala Asp Asn Ser Val Ser Val Gln Asp Asp Gly Arg Gly Ile Pro Thr
   65                  70                  75                  80
   Gly Ile His Pro Glu Glu Gly Val Ser Ala Ala Glu Val Ile Met Thr
                   85                  90                  95
   Val Leu His Ala Gly Gly Lys Phe Asp Asp Asn Ser Tyr Lys Val Ser
               100                 105                 110
   Gly Gly Leu His Gly Val Gly Val Ser Val Val Asn Ala Leu Ser Gln
           115                 120                 125
   Lys Leu Glu Leu Val Ile Gln Arg Glu Gly Lys Ile His Arg Gln Ile
       130                 135                 140
   Tyr Glu His Gly Val Pro Gln Ala Pro Leu Ala Val Thr Gly Glu Thr
   145                 150                 155                 160
   Glu Lys Thr Gly Thr Met Val Arg Phe Trp Pro Ser Leu Glu Thr Phe
                   165                 170                 175
   Thr Asn Val Thr Glu Phe Glu Tyr Glu Ile Leu Ala Lys Arg Leu Arg
               180                 185                 190
   Glu Leu Ser Phe Leu Asn Ser Gly Val Ser Ile Arg Leu Arg Asp Lys
           195                 200                 205
   Arg Asp Gly Lys Glu Asp His Phe His Tyr Glu Gly
       210                 215                 220
<210> 3
   <211> 660
   <212> DNA
   <213> Escherichia coli
<220>
   <221> misc_feature
   <223> Nucleotide sequence encoding GyrB
<400> 3
   atgtcgaatt cttatgactc ctccagtatc aaagtcctga aagggctgga tgcggtgcgt      60
   aagcgcccgg gtatgtatat cggcgacacg gatgacggca ccggtctgca ccacatggta     120
   ttcgaggtgg tagataacgc tatcgacgaa gcgctcgcgg gtcactgtaa agaaattatc     180
   gtcaccattc acgccgataa ctctgtctct gtacaggatg acgggcgcgg cattccgacc     240
   ggtattcacc cggaagaggg cgtatcggcg gcggaagtga tcatgaccgt tctgcacgca     300
   ggcggtaaat ttgacgataa ctcctataaa gtgtccggcg gtctgcacgg cgttggtgtt     360
   tcggtagtaa acgccctgtc gcaaaaactg gagctggtta tccagcgcga gggtaaaatt     420
   caccgtcaga tctacgaaca cggtgtaccg caggccccgc tggcggttac cggcgagact     480
   gaaaaaaccg gcaccatggt gcgtttctgg cccagcctcg aaaccttcac caatgtgacc     540
   gagttcgaat atgaaattct ggcgaaacgt ctgcgtgagt tgtcgttcct caactccggc     600
   gtttccattc gtctgcgcga caagcgcgac ggcaaagaag accacttcca ctatgaaggc     660
<210> 4
   <211> 522
   <212> DNA
   <213> Artificial Sequence
<220>
   <223> Description of sequence: FluA - flourescein (FluA_A45I_R95K_S114R)
<400> 4
   gacgtgtacc acgacggcgc ctgccccgaa gtgaagcccg tggacaactt cgactggtcc      60
   cagtaccacg gcaagtggtg ggaggtggcc aagtacccca gccccaacgg caagtatggc     120
   aagtgcggct ggattgagta cacccccgag ggcaagagcg tgaaggtgtc cagatacgac     180
   gtgatccacg gcaaagaata cttcatggaa ggcaccgcct accccgtggg cgacagcaag     240
   atcggcaaga tctaccacag ccggaccgtg ggcggctaca ccaagaagac cgtgttcaac     300
   gtgctgtcca ccgacaacaa gaactacatc atcggctacc gctgccgcta cgacgaggac     360
   aagaagggcc actgggacca cgtgtgggtg ctgtcccggt ccatggtgct gaccggcgag     420
   gccaagaccg ccgtggagaa ctacctgatc ggcagccccg tggtggacag ccagaaactg     480
   gtgtacagcg acttcagcga ggccgcctgc aaagtgaaca ac                        522
<210> 5
   <211> 175
   <212> PRT
   <213> Artificial Sequence
<220>
   <223> Description of sequence: FluA - flourescein (FluA_A45I_R95K_S114R)
<400> 5
   Met Asp Val Tyr His Asp Gly Ala Cys Pro Glu Val Lys Pro Val Asp
   1               5                   10                  15
   Asn Phe Asp Trp Ser Gln Tyr His Gly Lys Trp Trp Glu Val Ala Lys
               20                  25                  30
   Tyr Pro Ser Pro Asn Gly Lys Tyr Gly Lys Cys Gly Trp Ile Glu Tyr
           35                  40                  45
   Thr Pro Glu Gly Lys Ser Val Lys Val Ser Arg Tyr Asp Val Ile His
       50                  55                  60
   Gly Lys Glu Tyr Phe Met Glu Gly Thr Ala Tyr Pro Val Gly Asp Ser
   65                  70                  75                  80
   Lys Ile Gly Lys Ile Tyr His Ser Arg Thr Val Gly Gly Tyr Thr Lys
                   85                  90                  95
   Lys Thr Val Phe Asn Val Leu Ser Thr Asp Asn Lys Asn Tyr Ile Ile
               100                 105                 110
   Gly Tyr Arg Cys Arg Tyr Asp Glu Asp Lys Lys Gly His Trp Asp His
           115                 120                 125
   Val Trp Val Leu Ser Arg Ser Met Val Leu Thr Gly Glu Ala Lys Thr
       130                 135                 140
   Ala Val Glu Asn Tyr Leu Ile Gly Ser Pro Val Val Asp Ser Gln Lys
   145                 150                 155                 160
   Leu Val Tyr Ser Asp Phe Ser Glu Ala Ala Cys Lys Val Asn Asn
                   165                 170                 175
<210> 6
   <211> 552
   <212> DNA
   <213> Unknown
<220>
   <223> Description of sequence: DigA_16
<400> 6
   gacgtgtacc acgacggcgc ctgccccgaa gtgaagcccg tggacaactt cgactggtcc      60
   cagtaccacg gcaagtggtg gcaggtggcc gcttatcccg accacatcac caagtacggc     120
   aagtgcggct gggccgagta cacccccgag ggcaagagcg tgaaggtgtc ccggtacagc     180
   gtgatccacg gcaaagagta cttcagcgag ggcaccgcct accctgtggg cgacagcaag     240
   atcggcaaga tctaccacag ctacaccatc ggcggcgtga cccaggaggg cgtgttcaac     300
   gtgctgtcca ccgacaacaa gaactacatc atcggctact tttgcagata cgacgaggac     360
   aagaagggcc acatggactt ggtgtgggtg ctgtcccggt ccatggtgct gaccggcgag     420
   gccaagaccg ccgtggagaa ctacctgatc ggcagccccg tggtggacag ccagaaactg     480
   gtgtacagcg acttctccga ggccgcctgc aaagtgaaca acagcaactg gtcccacccc     540
   cagttcgaaa ag                                                         552
<210> 7
   <211> 185
   <212> PRT
   <213> Unknown
<220>
   <223> Description of sequence: DigA_16
<400> 7
   Met Asp Val Tyr His Asp Gly Ala Cys Pro Glu Val Lys Pro Val Asp
   1               5                   10                  15
   Asn Phe Asp Trp Ser Gln Tyr His Gly Lys Trp Trp Gln Val Ala Ala
               20                  25                  30
   Tyr Pro Asp His Ile Thr Lys Tyr Gly Lys Cys Gly Trp Ala Glu Tyr
           35                  40                  45
   Thr Pro Glu Gly Lys Ser Val Lys Val Ser Arg Tyr Ser Val Ile His
       50                  55                  60
   Gly Lys Glu Tyr Phe Ser Glu Gly Thr Ala Tyr Pro Val Gly Asp Ser
   65                  70                  75                  80
   Lys Ile Gly Lys Ile Tyr His Ser Tyr Thr Ile Gly Gly Val Thr Gln
                   85                  90                  95
   Glu Gly Val Phe Asn Val Leu Ser Thr Asp Asn Lys Asn Tyr Ile Ile
               100                 105                 110
   Gly Tyr Phe Cys Ser Tyr Asp Glu Asp Lys Lys Gly His Met Asp Leu
           115                 120                 125
   Val Trp Val Leu Ser Arg Ser Met Val Leu Thr Gly Glu Ala Lys Thr
       130                 135                 140
   Ala Val Glu Asn Tyr Leu Ile Gly Ser Pro Val Val Asp Ser Gln Lys
   145                 150                 155                 160
   Leu Val Tyr Ser Asp Phe Ser Glu Ala Ala Cys Lys Val Asn Asn Ser
                   165                 170                 175
   Asn Trp Ser His Pro Gln Phe Glu Lys
               180                 185
<210> 8
   <211> 1079
   <212> DNA
   <213> Unknown
<220>
   <223> Description of sequence: Salicyclic Acid Binding Protein 2 (SABP2)
<400> 8
   acgcggggaa agaaaagaaa ctaacaaggc ataaaattca aatgaaggaa ggaaaacact      60
   ttgttttagt acatggtgca tgccatggag gttggagttg gtacaagcta aagccactgc     120
   tagaagctgc aggccataag gttacagccc ttgatttagc agcttctggc actgatttga     180
   gaaaaataga ggagcttcgc acactttatg attatacttt gccattgatg gagttgatgg     240
   aatctctttc agcagatgag aaggttatat tagtggggca tagtcttggt ggtatgaatt     300
   tgggacttgc tatggaaaag tatccacaaa agatctatgc tgctgttttc ttggctgctt     360
   tcatgcctga ttctgttcac aactcctcct ttgttttgga acagtataat gagcggacgc      420
   cagccgagaa ttggttggat actcagtttt taccatatgg ttcccctgaa gagccactga      480
   catccatgtt ttttggccca aagttcttgg ctcacaagct ctaccagcta tgctctcctg      540
   aggatcttgc attagcatca tcattggtga gaccaagctc tttgtttatg gaagacctat      600
   cgaaggccaa gtatttcaca gatgaacggt ttggatcagt gaagagagtt tacattgtgt      660
   gcactgagga taaaggcata ccagaagaat tccagcgatg gcaaattgac aacattggtg      720
   tcactgaagc aatagagatt aaaggtgctg atcacatggc aatgctatgc gagccccaaa      780
   aactttgcgc ctctctcttg gaaattgccc ataaatacaa ctgatctcta cattatgtct      840
   tcgtctcatg tcaagatttt cagtgcatgc tgtaattttt ttctattttt cgaccggcgc      900
   ataactgtct ttgcctattt taaggattgc agtaatttca ctcttctagt gtggaaggct      960
   tccacataag gattgttctg tttctccatt caagtgtgtg ttatgttgag atacttaaac     1020
   cgtatcaatt cttgtaatga aacttcttct ttcctttttg aaaaaaaaaa aaaaaaaaa      1079
<210> 9
   <211> 260
   <212> PRT
   <213> Unknown
<220>
   <223> Description of sequence: Salicyclic Acid Binding Protein 2 (SABP2)
<400> 9
   Met Lys Glu Gly Lys His Phe Val Leu Val His Gly Ala Cys His Gly
   1               5                   10                  15
   Gly Trp Ser Trp Tyr Lys Leu Lys Pro Leu Leu Glu Ala Ala Gly His
               20                  25                  30
   Lys Val Thr Ala Leu Asp Leu Ala Ala Ser Gly Thr Asp Leu Arg Lys
           35                  40                  45
   Ile Glu Glu Leu Arg Thr Leu Tyr Asp Tyr Thr Leu Pro Leu Met Glu
       50                  55                  60
   Leu Met Glu Ser Leu Ser Ala Asp Glu Lys Val Ile Leu Val Gly His
   65                  70                  75                  80
   Ser Leu Gly Gly Met Asn Leu Gly Leu Ala Met Glu Lys Tyr Pro Gln
                   85                  90                  95
   Lys Ile Tyr Ala Ala Val Phe Leu Ala Ala Phe Met Pro Asp Ser Val
               100                 105                 110
   His Asn Ser Ser Phe Val Leu Glu Gln Tyr Asn Glu Arg Thr Pro Ala
           115                 120                 125
   Glu Asn Trp Leu Asp Thr Gln Phe Leu Pro Tyr Gly Ser Pro Glu Glu
       130                 135                 140
   Pro Leu Thr Ser Met Phe Phe Gly Pro Lys Phe Leu Ala His Lys Leu
   145                 150                 155                 160
   Tyr Gln Leu Cys Ser Pro Glu Asp Leu Ala Leu Ala Ser Ser Leu Val
                   165                 170                 175
   Arg Pro Ser Ser Leu Phe Met Glu Asp Leu Ser Lys Ala Lys Tyr Phe
               180                 185                 190
   Thr Asp Glu Arg Phe Gly Ser Val Lys Arg Val Tyr Ile Val Cys Thr
           195                 200                 205
   Glu Asp Lys Gly Ile Pro Glu Glu Phe Gln Arg Trp Gln Ile Asp Asn
       210                 215                 220
   Ile Gly Val Thr Glu Ala Ile Glu Ile Lys Gly Ala Asp His Met Ala
   225                 230                 235                 240
   Met Leu Cys Glu Pro Gln Lys Leu Cys Ala Ser Leu Leu Glu Ile Ala
                   245                 250                 255
   His Lys Tyr Asn
               260
<210> 10
   <211> 1079
   <212> DNA
   <213> Artificial Sequence
<220>
   <223> Description of sequence: Salicyclic Acid Binding Protein 2 (SABP2 S81A)
<400> 10
   acgcggggaa agaaaagaaa ctaacaaggc ataaaattca aatgaaggaa ggaaaacact      60
   ttgttttagt acatggtgca tgccatggag gttggagttg gtacaagcta aagccactgc     120
   tagaagctgc aggccataag gttacagccc ttgatttagc agcttctggc actgatttga     180
   gaaaaataga ggagcttcgc acactttatg attatacttt gccattgatg gagttgatgg     240
   aagctctttc agcagatgag aaggttatat tagtggggca tagtcttggt ggtatgaatt     300
   tgggacttgc tatggaaaag tatccacaaa agatctatgc tgctgttttc ttggctgctt     360
   tcatgcctga ttctgttcac aactcctcct ttgttttgga acagtataat gagcggacgc      420
   cagccgagaa ttggttggat actcagtttt taccatatgg ttcccctgaa gagccactga      480
   catccatgtt ttttggccca aagttcttgg ctcacaagct ctaccagcta tgctctcctg      540
   aggatcttgc attagcatca tcattggtga gaccaagctc tttgtttatg gaagacctat      600
   cgaaggccaa gtatttcaca gatgaacggt ttggatcagt gaagagagtt tacattgtgt      660
   gcactgagga taaaggcata ccagaagaat tccagcgatg gcaaattgac aacattggtg      720
   tcactgaagc aatagagatt aaaggtgctg atcacatggc aatgctatgc gagccccaaa      780
   aactttgcgc ctctctcttg gaaattgccc ataaatacaa ctgatctcta cattatgtct      840
   tcgtctcatg tcaagatttt cagtgcatgc tgtaattttt ttctattttt cgaccggcgc      900
   ataactgtct ttgcctattt taaggattgc agtaatttca ctcttctagt gtggaaggct      960
   tccacataag gattgttctg tttctccatt caagtgtgtg ttatgttgag atacttaaac     1020
   cgtatcaatt cttgtaatga aacttcttct ttcctttttg aaaaaaaaaa aaaaaaaaa      1079
<210> 11
   <211> 260
   <212> PRT
   <213> Artificial Sequence
<220>
   <223> Description of sequence: Salicyclic Acid Binding Protein 2 (SABP2 S81A)
<400> 11
   Met Lys Glu Gly Lys His Phe Val Leu Val His Gly Ala Cys His Gly
   1               5                   10                  15
   Gly Trp Ser Trp Tyr Lys Leu Lys Pro Leu Leu Glu Ala Ala Gly His
               20                  25                  30
   Lys Val Thr Ala Leu Asp Leu Ala Ala Ser Gly Thr Asp Leu Arg Lys
           35                  40                  45
   Ile Glu Glu Leu Arg Thr Leu Tyr Asp Tyr Thr Leu Pro Leu Met Glu
       50                  55                  60
   Leu Met Glu Ser Leu Ser Ala Asp Glu Lys Val Ile Leu Val Gly His
   65                  70                  75                  80
   Ala Leu Gly Gly Met Asn Leu Gly Leu Ala Met Glu Lys Tyr Pro Gln
                   85                  90                  95
   Lys Ile Tyr Ala Ala Val Phe Leu Ala Ala Phe Met Pro Asp Ser Val
               100                 105                 110
   His Asn Ser Ser Phe Val Leu Glu Gln Tyr Asn Glu Arg Thr Pro Ala
   115 120 125
   Glu Asn Trp Leu Asp Thr Gln Phe Leu Pro Tyr Gly Ser Pro Glu Glu
   130 135 140
   Pro Leu Thr Ser Met Phe Phe Gly Pro Lys Phe Leu Ala His Lys Leu
   145 150 155 160
   Tyr Gln Leu Cys Ser Pro Glu Asp Leu Ala Leu Ala Ser Ser Leu Val
   165 170 175
   Arg Pro Ser Ser Leu Phe Met Glu Asp Leu Ser Lys Ala Lys Tyr Phe
   180 185 190
   Thr Asp Glu Arg Phe Gly Ser Val Lys Arg Val Tyr Ile Val Cys Thr
   195 200 205
   Glu Asp Lys Gly Ile Pro Glu Glu Phe Gln Arg Trp Gln Ile Asp Asn
   210 215 220
   Ile Gly Val Thr Glu Ala Ile Glu Ile Lys Gly Ala Asp His Met Ala
   225 230 235 240
   Met Leu Cys Glu Pro Gln Lys Leu Cys Ala Ser Leu Leu Glu Ile Ala
   245 250 255
   His Lys Tyr Asn
   260
<210> 12
   <211> 1098
   <212> DNA
   <213> Unknown
<220>
   <223> Description of Sequence: Arabidopsis FK506 binding protein (FKBP42)
<400> 12
   atggatgaat ctctggagca tcaaactcaa acacatgacc aagagagcga aatagttact 60
   gaaggaagtg ccgttgtgca tagtgagcca tctcaagagg gtaatgttcc tcctaaagtt 120
   gatagtgaag ctgaggtctt ggatgagaaa gtcagtaagc agattataaa ggaaggtcac 180
   ggttccaaac catccaagta ctctacatgc tttttgcact acagggcatg gaccaaaaac 240
   tcgcagcaca aatttgagga tacatggcat gagcagcaac ctattgaatt ggttcttgga 300
   aaagagaaaa aagaactagc cggtttagcc atcggtgttg ctagcatgaa gtctggtgaa 360
   cgtgcgcttg tgcatgttgg ctgggaatta gcttatggga aagaaggaaa cttttctttt    420
   ccgaatgttc cacctatggc agacttgtta tatgaggtgg aagttattgg gtttgatgaa    480
   acaaaggagg gaaaagctcg cagtgatatg actgtagagg aaaggattgg tgcagcagac    540
   agaagaaaaa tggatgggaa ttctcttttt aaggaggaga aactggagga agccatgcaa    600
   cagtatgaaa tggccatagc atacatgggg gacgatttta tgtttcagct gtatgggaag    660
   taccaggata tggctttagc agttaaaaac ccatgccatc ttaacatagc agcttgcctc    720
   atcaaactaa aacgatacga tgaagcaatt ggtcactgca acattgtgtt gacagaagaa    780
   gagaaaaacc caaaagcact gttcagaaga gggaaagcaa aggcagagct aggacagatg    840
   gactcagcac gtgatgattt ccgaaaggca caaaagtatg ctcctgacga caaggcgatt    900
   agaagagagc tacgagcact tgcagagcaa gagaaagcct tgtaccaaaa gcagaaagaa    960
   atgtacaaag gaatattcaa agggaaagat gaaggtggtg ctaagtcaaa gagccttttt   1020
   tggttgatag tgttatggca atggtttgtt tcccttttct cccgtatctt tcgacgccac   1080
   agagttaaag cagattaa                                                 1098
<210> 13
   <211> 493
   <212> DNA
   <213> Unknown
<220>
   <223> Description of Sequence: Arabidopsis FK506 binding protein (FKBP42), aa 1-163
<400> 13
   atggatgaat ctctggagca tcaaactcaa acacatgacc aagagagcga aatagttact     60
   gaaggaagtg ccgttgtgca tagtgagcca tctcaagagg gtaatgttcc tcctaaagtt    120
   gatagtgaag ctgaggtctt ggatgagaaa gtcagtaagc agattataaa ggaaggtcac    180
   ggttccaaac catccaagta ctctacatgc tttttgcact acagggcatg gaccaaaaac    240
   tcgcagcaca aatttgagga tacatggcat gagcagcaac ctattgaatt ggttcttgga    300
   aaagagaaaa aagaactagc cggtttagcc atcggtgttg ctagcatgaa gtctggtgaa    360
   cgtgcgcttg tgcatgttgg ctgggaatta gcttatggga aagaaggaaa cttttctttt    420
   ccgaatgttc cacctatggc agacttgtta tatgaggtgg aagttattgg gtttgatgaa    480
   acaaaggagg taa                                                       493
<210> 14
   <211> 163
   <212> PRT
   <213> Unknown
<220>
   <223> Description of Sequence: Arabidopsis FK506 binding protein (FKBP42), aa 1-163
<400> 14
   Met Asp Glu Ser Leu Glu His Gln Thr Gln Thr His Asp Gln Glu Ser
   1               5                   10                  15
   Glu Ile Val Thr Glu Gly Ser Ala Val Val His Ser Glu Pro Ser Gln
               20                  25                  30
   Glu Gly Asn Val Pro Pro Lys Val Asp Ser Glu Ala Glu Val Leu Asp
           35                  40                  45
   Glu Lys Val Ser Lys Gln Ile Ile Lys Glu Gly His Gly Ser Lys Pro
       50                  55                  60
   Ser Lys Tyr Ser Thr Cys Phe Leu His Tyr Arg Ala Trp Thr Lys Asn
   65                  70                  75                  80
   Ser Gln His Lys Phe Glu Asp Thr Trp His Glu Gln Gln Pro Ile Glu
                   85                  90                  95
   Leu Val Leu Gly Lys Glu Lys Lys Glu Leu Ala Gly Leu Ala Ile Gly
               100                 105                 110
   Val Ala Ser Met Lys Ser Gly Glu Arg Ala Leu Val His Val Gly Trp
           115                 120                 125
   Glu Leu Ala Tyr Gly Lys Glu Gly Asn Phe Ser Phe Pro Asn Val Pro
       130                 135                 140
   Pro Met Ala Asp Leu Leu Tyr Glu Val Glu Val Ile Gly Phe Asp Glu
   145                 150                 155                 160
   Thr Lys Glu
<210> 15
   <211> 891
   <212> DNA
   <213> Unknown
<220>
   <223> Description of sequence: Arabidopsis multiresistance-like ABC transporter AtPGP1
<400> 15
   atgctcagat ccggtctggc ttcgttaatc gtcgatgtca atttgcggcg cacgttacgt      60
   ccatccccaa ccttttcttt tccggcgcat cttagccgtt gcattatcac ttcccgttac     120
   tcctcccgta catctctaag gtttccgatt caaatatctc gccaccaaca ccgtctatcc     180
   tacttttctt catcctcttc gtcggagcaa agcagaccaa cttcctcttc ccgaaacagt     240
   ttcagtggtc acggtcagct tgatagtgat gataattctt caccgcctcc gtcgcaatca     300
   tcttccaaag ttcttacatt gcctaccgta ttaacacttg gtcgtgtcgc cgccgtcccg     360
   cttctcgtcg caacctttta cgttgatagt tggtggggaa caactgctac aacaagcatt     420
   ttcattgcag cagccattac agactggctt gacggctatc ttgcccgcaa gatgaggtta     480
   ggttctgcgt ttggtgcctt tttggatcca gttgctgata agcttatggt tgcagctaca     540
   ttgattttac tgtgtacaaa acctatccaa gttgctgaat taggaccact tccatggtta     600
   ttgaccgtac cttctattgc aatcattggt agggagatta ctatgtccgc agtaagagaa     660
   tgggctgcat ctcaaaatgg aaagctttta gaggcagttg ctgtaaataa cttgggcaag     720
   tggaaaaccg ccacgcagat gacagcacta accatacttc ttgcaagccg agatagcaat     780
   gttggatggc tcgtagcttc aggtgctggc ttgctttatg tatcagcagg actatctgtt     840
   tggtctttag ccgtttatat gaggaagata tggaaagtac taatgaagta g              891
<210> 16
   <211> 296
   <212> PRT
   <213> Unknown
<220>
   <223> Description of sequence: Arabidopsis multiresistance-like ABC transporter AtPGP1
<400> 16
   Met Leu Arg Ser Gly Leu Ala Ser Leu Ile Val Asp Val Asn Leu Arg
   1               5                   10                  15
   Arg Thr Leu Arg Pro Ser Pro Thr Phe Ser Phe Pro Ala His Leu Ser
               20                  25                  30
   Arg Cys Ile Ile Thr Ser Arg Tyr Ser Ser Arg Thr Ser Leu Arg Phe
           35                  40                  45
   Pro Ile Gln Ile Ser Arg His Gln His Arg Leu Ser Tyr Phe Ser Ser
       50                  55                  60
   Ser Ser Ser Ser Glu Gln Ser Arg Pro Thr Ser Ser Ser Arg Asn Ser
   65                  70                  75                  80
   Phe Ser Gly His Gly Gln Leu Asp Ser Asp Asp Asn Ser Ser Pro Pro
                   85                  90                  95
   Pro Ser Gln Ser Ser Ser Lys Val Leu Thr Leu Pro Thr Val Leu Thr
               100                 105                 110
   Leu Gly Arg Val Ala Ala Val Pro Leu Leu Val Ala Thr Phe Tyr Val
           115                 120                 125
   Asp Ser Trp Trp Gly Thr Thr Ala Thr Thr Ser Ile Phe Ile Ala Ala
       130                 135                 140
   Ala Ile Thr Asp Trp Leu Asp Gly Tyr Leu Ala Arg Lys Met Arg Leu
   145                 150                 155                 160
   Gly Ser Ala Phe Gly Ala Phe Leu Asp Pro Val Ala Asp Lys Leu Met
                   165                 170                 175
   Val Ala Ala Thr Leu Ile Leu Leu Cys Thr Lys Pro Ile Gln Val Ala
               180                 185                 190
   Glu Leu Gly Pro Leu Pro Trp Leu Leu Thr Val Pro Ser Ile Ala Ile
           195                 200                 205
   Ile Gly Arg Glu Ile Thr Met Ser Ala Val Arg Glu Trp Ala Ala Ser
       210                 215                 220
   Gln Asn Gly Lys Leu Leu Glu Ala Val Ala Val Asn Asn Leu Gly Lys
   225                 230                 235                 240
   Trp Lys Thr Ala Thr Gln Met Thr Ala Leu Thr Ile Leu Leu Ala Ser
                   245                 250                 255
   Arg Asp Ser Asn Val Gly Trp Leu Val Ala Ser Gly Ala Gly Leu Leu
               260                 265                 270
   Tyr Val Ser Ala Gly Leu Ser Val Trp Ser Leu Ala Val Tyr Met Arg
           275                 280                 285
   Lys Ile Trp Lys Val Leu Met Lys
       290                 295
<210> 17
   <211> 920
   <212> DNA
   <213> Unknown
<220>
   <223> Description of sequence: Arabidopsis multiresistance-like ABC transporter AtPGP1 (aa 980 - 1286)
<400> 17
   cactggctcc tgatttcatc aaaggtggtc aagctatgcg gtctgttttc gaacttcttg      60
   accggaaaac cgagattgaa cctgatgatc ctgataccac cccggtccca gaccggttac     120
   gtggtgaagt cgagctcaaa catatcgatt tctcttaccc ttcaaggcca gacatccaga      180
   ttttccgtga ccttagcctt cgtgctagag ctggcaaaac cctagctctt gtgggtccaa      240
   gcgggtgcgg aaaaagctca gttatctccc tcatccagag attctacgaa ccttcctcag      300
   gccgagtcat gatcgacggg aaagacataa ggaaatacaa cctgaaagcc ataaggaaac      360
   acatagccat agtccctcaa gagccgtgct tgttcggaac taccatttac gaaaacattg      420
   catatggaca tgaatgtgcg accgaagcag agatcataca agccgcgact ctagccagtg      480
   cgcacaaatt catatccgcg ctaccagaag gttacaaaac gtatgttggc gagagaggcg      540
   ttcagctctc gggaggacag aaacagagga tcgcgatcgc acgtgccctc gtgaggaaag      600
   cagagatcat gctgcttgac gaggctacaa gcgctcttga tgcagagtcc gagagatcag      660
   tccaagaagc attagaccag gcttgctctg gtagaacatc aatagtcgtg gctcataggc      720
   tatctacaat caggaacgca cacgtgatcg ctgtcatcga tgatggaaaa gtggctgaac      780
   aaggatcgca ttcgcatctt ctcaagaacc atcctgatgg aatctacgcg cgaatgatac      840
   agttgcaaag atttacgcat acacaagtga ttggtatgac gtcaggttca agttctaggg      900
   ttaaggaaga tgatgcttag                                                  920
<210> 18
   <211> 306
   <212> PRT
   <213> Unknown
<220>
   <223> Description of sequence: Arabidopsis multiresistance-like ABC transporter AtPGP1 (aa 980 - 1286)
<400> 18
   Thr Leu Ala Pro Asp Phe Ile Lys Gly Gly Gln Ala Met Arg Ser Val
   1               5                   10                  15
   Phe Glu Leu Leu Asp Arg Lys Thr Glu Ile Glu Pro Asp Asp Pro Asp
               20                  25                  30
   Thr Thr Pro Val Pro Asp Arg Leu Arg Gly Glu Val Glu Leu Lys His
           35                  40                  45
   Ile Asp Phe Ser Tyr Pro Ser Arg Pro Asp Ile Gln Ile Phe Arg Asp
       50                  55                  60
   Leu Ser Leu Arg Ala Arg Ala Gly Lys Thr Leu Ala Leu Val Gly Pro
   65                  70                  75                  80
   Ser Gly Cys Gly Lys Ser Ser Val Ile Ser Leu Ile Gln Arg Phe Tyr
                   85                  90                  95
   Glu Pro Ser Ser Gly Arg Val Met Ile Asp Gly Lys Asp Ile Arg Lys
               100                 105                 110
   Tyr Asn Leu Lys Ala Ile Arg Lys His Ile Ala Ile Val Pro Gln Glu
           115                 120                 125
   Pro Cys Leu Phe Gly Thr Thr Ile Tyr Glu Asn Ile Ala Tyr Gly His
       130                 135                 140
   Glu Cys Ala Thr Glu Ala Glu Ile Ile Gln Ala Ala Thr Leu Ala Ser
   145                 150                 155                 160
   Ala His Lys Phe Ile Ser Ala Leu Pro Glu Gly Tyr Lys Thr Tyr Val
                   165                 170                 175
   Gly Glu Arg Gly Val Gln Leu Ser Gly Gly Gln Lys Gln Arg Ile Ala
               180                 185                 190
   Ile Ala Arg Ala Leu Val Arg Lys Ala Glu Ile Met Leu Leu Asp Glu
           195                 200                 205
   Ala Thr Ser Ala Leu Asp Ala Glu Ser Glu Arg Ser Val Gln Glu Ala
       210                 215                 220
   Leu Asp Gln Ala Cys Ser Gly Arg Thr Ser Ile Val Val Ala His Arg
   225                 230                 235                 240
   Leu Ser Thr Ile Arg Asn Ala His Val Ile Ala Val Ile Asp Asp Gly
                   245                 250                 255
   Lys Val Ala Glu Gln Gly Ser His Ser His Leu Leu Lys Asn His Pro
               260                 265                 270
   Asp Gly Ile Tyr Ala Arg Met Ile Gln Leu Gln Arg Phe Thr His Thr
           275                 280                 285
   Gln Val Ile Gly Met Thr Ser Gly Ser Ser Ser Arg Val Lys Glu Asp
       290                 295                 300
   Asp Ala
   305
<210> 19
   <211> 264
   <212> PRT
   <213> Artificial Sequence
<220>
   <223> Description of sequence: scFv fragment directed against fluorescein and FITC-E2 after removal of the periplasmic signal sequence
<400> 19
   Gln Val Gln Leu Val Glu Ser Gly Gly Asn Leu Val Gln Pro Gly Gly
   1               5                   10                  15
   Ser Leu Arg Leu Ser Cys Ala Ala Ser Gly Phe Thr Phe Gly Ser Phe
               20                  25                  30
   Ala Met Ser Trp Val Arg Gln Ala Pro Gly Gly Gly Leu Glu Trp Val
           35                  40                  45
   Ala Gly Leu Ser Ala Arg Ser Ser Leu Thr His Tyr Ala Asp Ser Val
       50                  55                  60
   Lys Gly Arg Phe Thr Ile Ser Arg Asp Asn Ala Lys Asn Ser Val Tyr
   65                  70                  75                  80
   Leu Gln Met Asn Ser Leu Arg Val Glu Asp Thr Ala Val Tyr Tyr Cys
                   85                  90                  95
   Ala Arg Arg Ser Tyr Asp Ser Ser Gly Tyr Trp Gly His Phe Tyr Ser
               100                 105                 110
   Tyr Met Asp Val Trp Gly Gln Gly Thr Leu Val Thr Val Ser Ser Gly
           115                 120                 125
   Gly Gly Gly Ser Gly Gly Gly Gly Ser Gly Gly Gly Gly Ser Gln Ser
       130                 135                 140
   Val Leu Thr Gln Pro Ser Ser Val Ser Ala Ala Pro Gly Gln Lys Val
   145                 150                 155                 160
   Thr Ile Ser Cys Ser Gly Ser Thr Ser Asn Ile Gly Asn Ala Tyr Val
                   165                 170                 175
   Ser Trp Tyr Gln Gln His Pro Gly Lys Ala Pro Lys Leu Met Ile Tyr
               180                 185                 190
   Asp Val Ser Lys Arg Pro Ser Gly Val Pro Asp Arg Phe Ser Gly Ser
           195                 200                 205
   Lys Ser Gly Asn Ser Ala Ser Leu Asp Ile Ser Gly Leu Gln Ser Glu
       210                 215                 220
   Asp Glu Ala Asp Tyr Tyr Cys Ala Ala Trp Asp Asp Ser Leu Ser Glu
   225                 230                 235                 240
   Phe Leu Phe Gly Thr Gly Thr Lys Leu Thr Val Leu Gly Ala Ser Gly
                   245                 250                 255
   Ala Asp His His His His His His
               260

## Description

### Field of invention

The present invention relates to the preparation and use of reversibly PEGylated nanocarriers. The reversibly PEGgylated nanocarrier system comprises an inner core comprising or consisting of a nanoparticle and/or at least one pharmaceutical compound; optionally at least one lipid bilayer on the surface of the inner core; a first ligand bound to the surface of said inner core, or if present, to the surface of the at least one lipid bilayer; a second ligand bound to the first ligand, the second ligand being covalently bound to poly(ethylene glycol) or a derivative thereof. The reversibly PEGgylated nanocarrier system is as defined in the claims and characterized in that binding of the second ligand to the first ligand is non-covalent and reversible. Pharmaceutical compositions and use of such compounds and compositions in the field of e.g. cancer therapy, gene therapy, inflammatory diseases and pain therapy are also disclosed.

### Background of invention

Pharmaceutical nanoparticles or nanocarriers, such as liposomes, micelles, etc., are an essential component in the emerging field of nanomedical imaging and therapy. Unmodified pharmaceutical nanoparticles or nanocarriers are typically rapidly cleared from the circulation by the liver, spleen or bone marrow, and nonspecific binding to non-targeted or non-diseased areas may occur.

In the field of such pharmaceutical nanoparticles/nanocarriers, surface coating with polyethylene glycol (PEGylation) has therefore been established as the most popular and elaborated method to prepare drug delivery systems which provide for a prolonged circulation time of the nanoparticles/nanocarriers in the blood. PEGylation protects these carriers from an immediate uptake by the reticuloendothelial (RE) system (macrophage uptake). PEGylated nanocarriers generally accumulate in the liver a half to a third of the amount of non-PEGylated nanocarriers and most importantly demonstrate e.g. higher tumor accumulation versus background.

Such prolonged circulation is often required to provide sufficient time for effective accumulation of drug-loaded (drug, siRNA, pDNA) nanocarriers in target organs or tissues. In this context, PEGylation improves the stability of nanoparticles.

PEG is inexpensive, versatile and is FDA approved for many applications. However, PEGylation of such pharmaceutical nanoparticles/nanocarriers also has some severe drawbacks. Particularly, the high stabilization of nanoparticles/nanocarriers with PEG leads to prolonged circulation times but most often also results in poor endosomal escape via membrane fusion and in degradation of cargos in lysosomes, digestive compartments, etc. This problem, also referred to as the 'PEG dilemma', may significantly hamper efficiency of the use of PEG and PEGylated nanoparticles/nanocarriers for drug delivery or in gene delivery.

Passive targeting in cancer tissues is discussed in Allen & Cullis (see Theresa M. Allen, Pieter R. Cullis, Advanced Drug Delivery Reviews 65 (2013) 36-48). As described by Allen and Cullis (2013) "Long circulating (PEGylated) liposomes were shown to have extensive accumulation in Kaposi's sarcoma and head and neck cancers, with intermediate accumulation in lung cancer and lower accumulation in breast cancer in an initial study using small numbers of patients. However, to draw specific conclusions about the relationship between tumor types, stage of tumor development and liposome accumulation these studies need to be repeated with larger cohorts. The extent of liposomal accumulation may be related, at least in part, to the degree of angiogenesis of the tissues." ... "In clinical applications, liposomal drugs have been proven to be most useful for their ability to "passively" accumulate at sites of increased vasculature permeability, when their average diameter is in the ultrafilterable range (200 nm in diameter), and for their ability to reduce the side effects of the encapsulated drugs relative to free drugs. This has resulted in an overall increase in therapeutic index, which measures efficacy over toxicity."

Hatakeyama et al. (see Hatakeyama et al., Advanced Drug Delivery Reviews 63 (2011)) also found that PEGylation is a useful tool but hampered by slow or even minimal endosomal escape of the carriers. Hatakeyama et al. (2011) noted that "despite the great success of delivering chemotherapeutic drugs to tumors using PEGylated nanoparticles via passive targeting based on the EPR effect, PEGylation hampers in vivo applications of nanoparticles as gene carriers of pDNA and siRNA to tumor delivery. For pDNA or siRNA to be therapeutically efficacious, gene carriers must deliver their cargos to the appropriate intracellular compartment where pDNA or siRNA functions, that is, the nucleus or the cytosol. They must pass through the cell membrane and undergo sequential endosome/lysosome degradation, unlike chemotherapeutic drugs such as doxorubicin, which spontaneously accumulate in the nucleus due to their capability to intercalate into double-stranded DNA. The surface aqueous phase formed by the PEG moiety inhibits the interaction of the gene carrier with the tumor cell surface. As a result, cellular uptake is nil or minimal."

Schnyder et al. (Biochem. J. (2004), 377, 61-67) demonstrated the targeting of skeletal muscle in vitro using biotinylated immunoliposomes. Schnyder et al (2004) is silent on the use of reversible PEGylation of nanocarriers using single chain antibody fragments against Biotin or Fluorescein.

EP 2 455 104 A1 concerns Bio-functionalized stimulus responsive dissolvable PEG hydrogels. EP 2 455 104 A1 is silent on Reversibly PEGylated nanocarrier system comprising: an inner core comprising a nanoparticle and/or at least one pharmaceutical compound; a first ligand bound to the surface of said inner core; a second ligand bound to the first ligand, the second ligand being furthermore covalently bound to poly(ethylene glycol) or a derivative thereof, characterized in that binding of the second ligand to the first ligand is non-covalent and is reversible. EP 2 455 104 A1 is also silent on the specific use of the reversible PEGylation of such nanocarriers using single chain antibody fragments against Biotin or Fluorescein.

In sum, PEGylation is a useful method for achieving a longer circulation time for delivery to a tumor via the enhanced permeability and retention effect. However, PEGylation strongly inhibits cellular uptake and endosomal escape, which results in significant loss of activity for the delivery system. For successful drug delivery for cancer treatment, the crucial issue associated with the use of PEG, the 'PEG dilemma', must be addressed successfully.

Several attempts have been carried out meanwhile to provide PEGylated nanoparticles/nanocarriers which address the 'PEG dilemma'. Such attempts comprise both passive targeting but also a triggered release of PEG from PEGylated nanoparticles/nanocarriers by providing a "cleavable" PEG system.

Allen & Cullis (2013) reviewed currently available methods which allow releasing the target of such PEGylated nanoparticles/nanocarriers either upon a local or an external stimulus. In this context, two main types of triggers have been explored: Remote triggers such as heat, ultrasound or light, and local triggers that are intrinsic to the disease site or cellular organelles such as enzymes or pH changes. Examples include temperature dependent PEG cleavage (local, e.g. increased temperature in inflamed or neoplastic tissue, or externally applied), pH dependent PEG cleavage (local, e.g. low pH in tumor tissue), enzymatic cleavage by system specific (local) proteases (phospholipases, alkaline phosphatase, metalloproteinases); s.a. patent CN: metalloproteinase secreted by tumor cells, ultrasonic release (externally applied; remote trigger), light or magnetically triggered (externally applied; remote trigger), combinations of the above, or a combination of such triggers with targeting agents (e.g. Hyperthermia-triggered & Her2 -targeted intracellular doxorubicin delivery).

However, even though such triggers appeared to provide a good alternative to shed PEG from nanocarriers to facilitate endosomal escape, such approaches have been disappointing in practice as also documented by Allen & Cullis (2013). Local stimuli intrinsic to the tumor, such as pH or temperature changes, are neither switchable on demand, nor are they efficient. If switchable systems are used, these are often hindered by the PEG shell (e.g. when using proteases as a trigger), they are challenging to administer in deep-tissue regions (e.g. when using light, temperature or ultrasound as a trigger) and in case the liposomes are destroyed, release occurs outside the cells (e.g. when using temperature or ultrasound as a trigger). Furthermore, metastatic disease is a common cause of death in advanced cancer, but small and potentially unidentified metastatic tumors are not accessible via remote triggers. Hence, applications for remote triggered formulations should be carefully chosen and would include applications such as locally advanced disease and cancer where tissue sparing is preferred for reasons of preserving quality of life.

Hence, no suitable applications have yet been presented in the prior art for efficiently overcoming the PEG dilemma, based on the manipulation of intracellular trafficking of cellular uptake and endosomal release using functional devices such as specific ligands or cleavable PEG systems.

It is therefore the object of the present invention to provide a PEGylated nanocarrier system which allows to efficiently aid transport to and preferably also allows to release its target at the site of treatment and thereby at least in part overcomes the PEG dilemma as described before.

For a complete understanding of the present invention and the advantages thereof, reference is made to the following detailed description taken in conjunction with the accompanying figures and examples.

### Summary of invention

The present invention pertains to a reversibly PEGylated nanocarrier system comprising: an inner core comprising a nanoparticle and/or at least one pharmaceutical compound; optionally at least one lipid bilayer on the surface of the inner core; a first ligand bound to the surface of said inner core, or if present, to the surface of the at least one lipid bilayer; a second ligand bound to the first ligand, the second ligand being covalently bound to poly(ethylene glycol) or a derivative thereof, characterized in that binding of the second ligand to the first ligand is non-covalent and reversible. Herein, the first ligand and the second ligand are selected from the following first ligand/second ligand combinations:

| **First ligand** | **Second ligand** |
|---|---|
| Fluorescein, FITC | scFv fragment FITC-E2 |
| Fluorescein, FITC | scFv fragment FITC-E2 according to SEQ ID NO: 1 or 19 or a sequence showing at least 80% identity to the sequence of SEQ ID NO: 1 or 19 |
| Biotin | anti-Biotin scFv Fragment |

Such reversibility can be achieved *in vitro* and *in vivo* and is preferably triggered upon addition of a non-bound excess amount of the first ligand or the second ligand to the reversibly PEGylated nanocarrier system, or by addition of any other competitively acting ligand, leading to a competitive release of the second ligand and hence of the PEGylation. Further preferred aspects are outlined below.

According to a further embodiment, the present invention provides a pharmaceutical composition comprising the inventive reversibly PEGylated nanocarrier system as described herein and optionally a pharmaceutically acceptable carrier and/or vehicle.

According to another embodiment, the present invention discloses the use of the inventive reversibly PEGylated nanocarrier system as a medicament.

Likewise, according to a further embodiment the present invention discloses the use of the inventive reversibly PEGylated nanocarrier system in cancer therapy or in gene therapy. Further preferred aspects of the inventive embodiments are outlined below and in dependent claims.

### Brief description of Figures

The following Figures shall illustrate the above described invention in further detail and are not intended to limit the scope of the claims thereto.

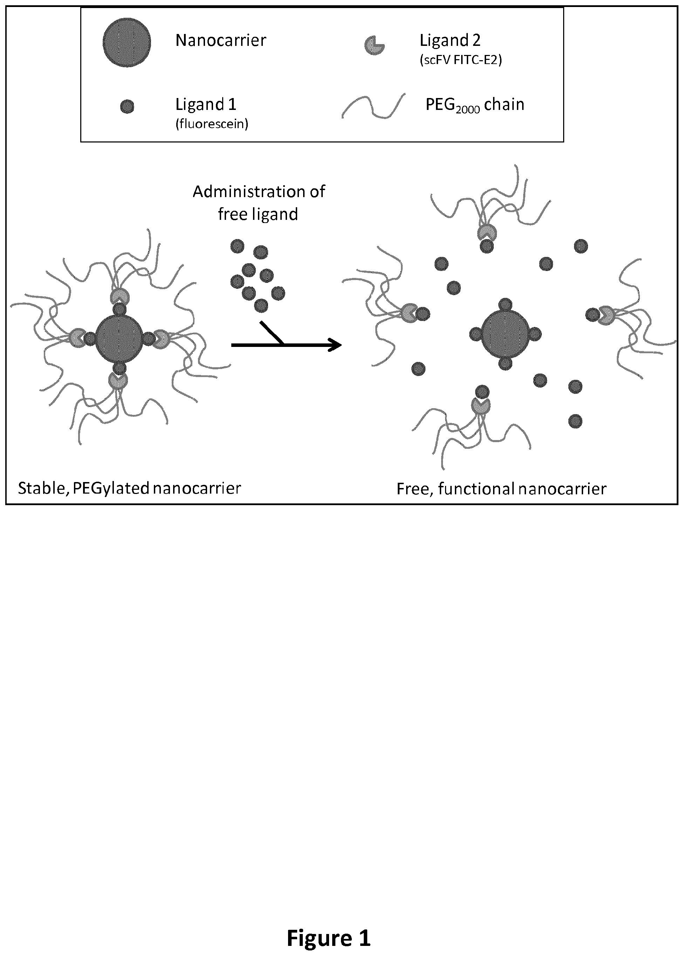

- **Figure 1:**: shows the setup of the inventive reversibly PEGylated nanocarrier system. Nanocarriers carrying a first ligand, e.g. fluorescein, on the surface are bound by PEGylated FITC-E2 (scFv) as a specific FITC binding protein with high specificity, the FITC-E2 (scFv) acting as a second ligand as defined herein to which PEG is covalently bound. This set up leads to a protection of the nanoparticle and/or pharmaceutical compound (inner core) and a controlled release. Controlled release can be achieved by addition of an excess of free (first) ligand (e.g. again fluorescein, or any other competitively acting ligand), wherein the competing effects lead to a disruption of the interaction between the first and second ligand, e.g. PEGylated scFv and the fluorescein bound on the nanocarrier. The nanocarrier can be also provided with at least one lipid bilayer around the inner core, wherein the first ligand is then preferably bound to the lipid bilayer.

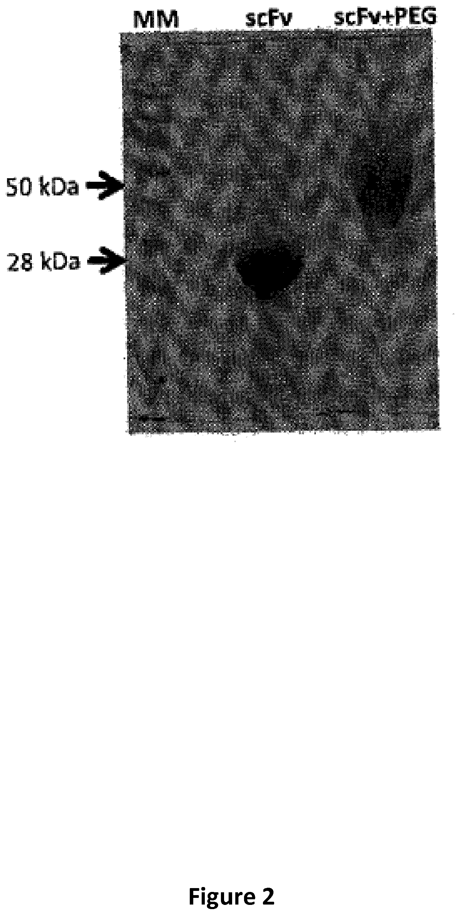

- **Figure 2:**: shows a 12% SDS PAGE of the FITC-E2. MM represents the molecular marker. scFv represents FITC-E2, scFv+PEG represents the single short chain fragment FITC-E2 modified with PEG (PEGylated; In lane "scFV" 20 µl FITC-E2 (18mg/ml) were loaded in the corresponding lane. In the lane "scFv+PEG" scFv was reacted with 6x excess of NHS-mPEG2000 with the same loading conditions as for scFv.

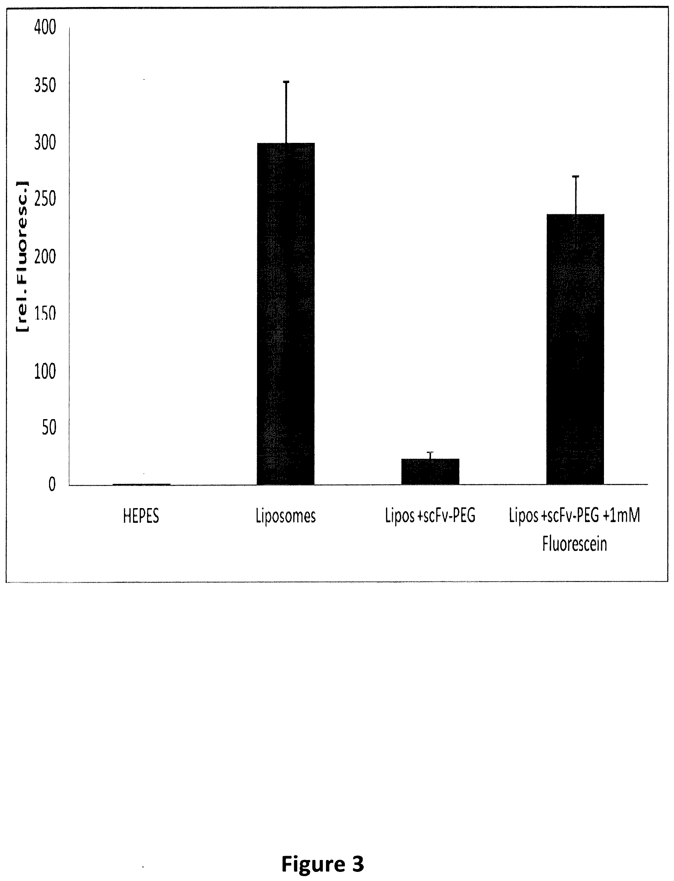

- **Figure 3:**: shows a test of the functionality of the inventive reversibly PEGylated nanocarrier system on THP-1 cells. For this purpose, THP-1 cells were incubated 90 minutes with different formulations of liposomes (liposomes, liposomes + scFv-PEG or liposomes + scFv-PEG + fluorescein) or just buffer (HEPES). Cells were washed three times with PBS 37 °C and then detached with PBS + 2 mM EDTA. The resulting fluorescence of THP-1 cells was afterwards determined with a flow cytometer.
As could be seen in Figure 3, the fluorescence of the cells correlates with the uptake of DiD stained liposomes. The determined uptake rate is comparable with the figures obtained with "normal" PEGylated liposomes (see Blume G, Cevc G (1990) Biochim Biophys Acta, 1029(1):91-7), and is nearly 100% reversible after addition of fluorescein to a final concentration of 1 mM.

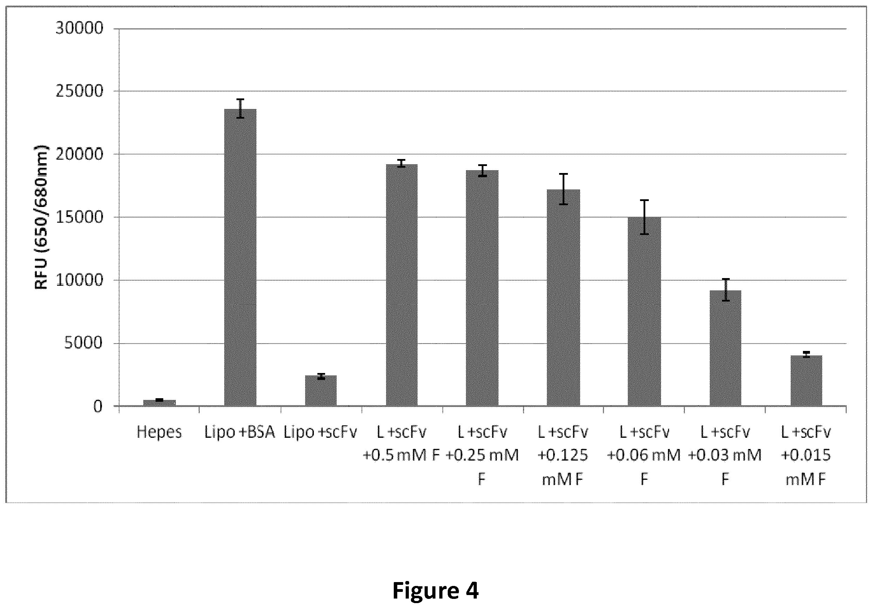

- **Figure 4:**: shows fluorescein-dependent scFv-PEG de-shielding of the nanocarrier (liposome) in the presence of THP-1 cells. THP-1 cells were incubated 90 minutes with bare liposomes or liposomes with scFv-PEG and different concentrations of fluorescein (500 µM, 250 µM, 125 µM, 60 µM, 30 µM, 15 µM and just HEPES-buffer). Cells were washed three times with PBS 37 °C and then lysed with HEPES 2% Triton-X. The uptake was quantified by measuring the DiD fluorescence, which the liposomal membranes were stained with (L: liposomes; F: fluorescein).

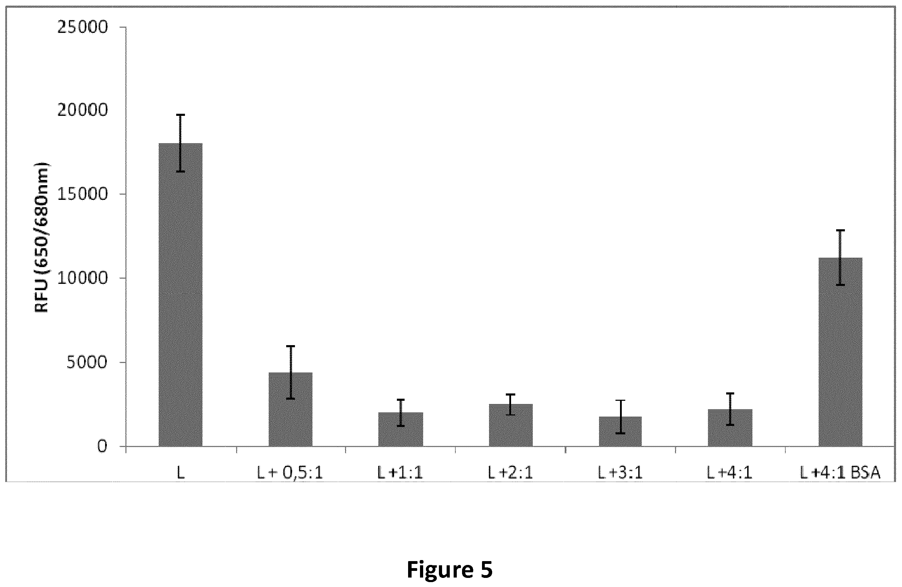

- **Figure 5:**: shows a test of different concentration of scFv-PEG to provide the scFv-PEG shell protection to the nanocarrier (liposome) in the presence of THP-1 cells. THP-1 cells were incubated 90 minutes with bare liposomes or liposomes with different scFv-PEG concentrations (5mol%-PE-FITC in liposomal membrane; molar ratio scFv-PEG:PE-FITC: 1:2; 1:1; 2:1, 4:1, or BSA 4:1). Cells were washed three times with PBS 37 °C and then lysed with HEPES 2% Triton-X. The uptake was quantified by measuring the DiD fluorescence, which the liposomal membranes were stained with (L: liposomes).

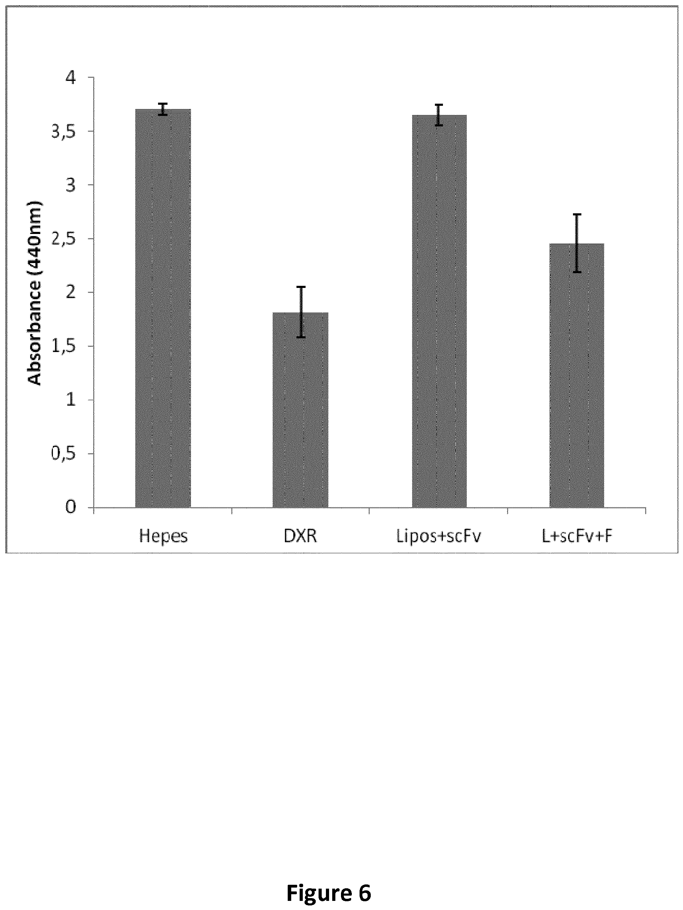

- **Figure 6:**: shows a test of the functionality of the inventive reversibly PEGylated nanocarrier system in a CHO-cell proliferation assay. For this purpose, CHO cells were incubated 4 hours with doxorubicin or doxorubicin loaded liposomes in combination with the protective shell with or without the addition of fluorescein (HEPES, Doxorubicin, Doxorubicin liposomes + scFv-PEG or doxorubicin liposomes + scFv-PEG + fluorescein). Cells were washed three times with PBS 37 °C and then further cultivated for 24 h. To quantify the cell proliferation a WST-1 assay (Roche) was performed.

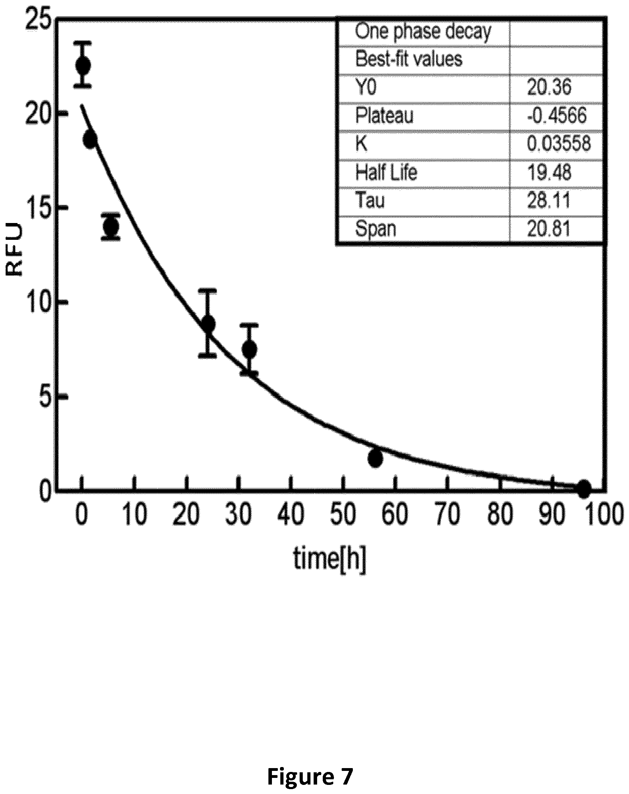

- **Figure 7:**: Plasma half-life of doxorubicin loaded into liposomes with PEGylated scFv in *Rattus norvegicus* (dosage 3.75 mg/rat). A prolonged half-life (19.5 h) of the protected liposomal doxorubicin can be seen compared to literature data for free doxorubicin (<1h) or liposomal, unprotected, doxorubicin (1-2h).

### Detailed description

The object underlying the present invention has been solved by the attached independent claims. Further preferred embodiments are defined in the dependent claims as attached. However, it should be appreciated that the various aspects of the disclosure herein are illustrative of the specific ways to make and use the invention and do not limit the scope of the invention when taken into consideration with the claims and the following detailed description. It will also be appreciated that features from one aspect of the invention may be combined with features from another aspect of the invention.

A first embodiment of the present invention is directed to a reversibly PEGgylated nanocarrier system. The inventive system comprises:
- an inner core comprising or consisting of a nanoparticle and/or at least one pharmaceutical compound;
- optionally at least one lipid bilayer on the surface of the inner core;
- a first ligand bound to the surface of said inner core, or if present, to the surface of the at least one lipid bilayer;
- a second ligand bound to the first ligand, the second ligand being covalently bound to poly(ethylene glycol) or a derivative thereof,

characterized in that binding of the second ligand to the first ligand is non-covalent and reversible. Reversibility is preferably triggered upon addition of a non-bound excess amount of the first ligand or the second ligand to the reversibly PEGgylated nanocarrier system or by addition of any other competitively acting ligand.

The inventive reversibly PEGgylated nanocarrier system provides a multiplicity of advantages over systems of the art.

Generally, the inventive reversibly PEGgylated nanocarrier system provides a PEGylated carrier which efficiently accumulates at least passively in the selected target tissue. Upon administration of an excess of free ligand (remote trigger) the cargo is more easily released. The inventive reversibly PEGgylated nanocarrier system also allows providing a (targeted or passive) interaction with surrounding tissue since the inventive system is a highly modular system and can be adopted to a variety of pharmaceutical compositions and be selected to target selectively a certain target region. In the context of the inventive reversibly PEGgylated nanocarrier system a large variety of nanocarriers are applicable, e.g. liposomal Doxorubicin, etc., wherein the components of the herein described first ligand - second ligand system are highly exchangeable. Particularly the first ligand - second ligand systems outlined exemplary herein, e.g. second ligand FITC-E2 scFv and first ligand fluorescein, are safe for humans. The interaction of the components of these selected first ligand - second ligand systems nevertheless is highly specific and reversible. Since the second ligand is covalently bound to PEG, the inventive nanocarrier system is effectively PEGylated via its interaction between the first and the second ligand, but at the same time allows to efficiently detach its PEGylation at the target region or any desired location upon adding a specific trigger as described herein and thereby to release its cargo.

Advantageously, the interaction between the components of the selected first ligand - second ligand systems can be disrupted again by competing the binding with addition of e.g. free first ligand or second ligand or any other competitively acting ligand, which then represents an externally applied, effective trigger. Such a trigger can be administered at any desired, most beneficial point of time. Such a trigger is highly independent of local tissue specific factors and may even allow targeting small metastatic tumors. The inventive PEGylation system can therefore be released on demand, which results in increased drug specificity and hence also in reduced drug-related side effects, reduced adverse body reactions against PEGylated particles due to shortened circulation after triggered release, etc. Furthermore, different and preferably optimized rates of PEGylation could be adjusted and the preferential cargo can be selected from a variety of small molecule anticancer drugs either individually or in combination.

In the context of the present invention the term "PEGylated" refers to the modification of a herein described nanocarrier system with a poly(ethylene glycol) (PEG) or a derivative thereof, preferably using the herein described system of first and second ligands and their combinations as outlined below.

In the context of the present invention a "reversibly PEGylated nanocarrier system" is preferably to be understood as a PEGylated nanocarrier system, wherein poly(ethylene glycol) (PEG) or a derivative thereof has been attached to the herein described nanocarrier system such that the attachment is reversible. Such a reversible attachment typically occurs via a non-covalent bond, such as via electrostatic interactions, van-der-Waals interactions, etc., or any further interactions to be subsumed as a non-covalent bond. The reversible attachment in the context of the present invention is specifically based on the (non-covalent) interaction between the corresponding first and second ligands as described herein. Since the second ligand is covalently bound to PEG or a derivative thereof, the reversibility and the detachment of PEG or a derivative thereof can be induced upon addition of a non-bound excess amount of the first ligand or of the second ligand to the reversibly PEGgylated nanocarrier system, or by addition of any other competitively acting ligand. This addition leads to a competitive release of the interaction between the first and the second ligand and hence to a release of the PEGylation.

In the context of the present invention the term "covalent", e.g. in terms "covalent bond" or "covalent binding", preferably means any covalent bond known to a skilled person. A covalent bond as defined herein may be formed by chemical reactions known to a skilled person, e.g. by adding chemical groups able to react with each other or a functional group and thereby forming a chemical bond. Examples of such coupling reactions are known to a skilled person and reviewed e.g. in the publication "Bioanalytik", Springer Verlag, Lottspeich, Friedrich; Engels, Joachim W. (publisher), chapter 7, Chemische Modifikation von Proteinen und Proteinkomplexen.

Likewise, in the context of the present invention a "nanocarrier system" is preferably to be understood as a transport system for nanoparticles and/or at least one or more pharmaceutical compound(s), wherein the nanoparticles in the context of the present invention are provided such as to exhibit a size in the range of several nanometers and preferably of less than 1 µm, as further defined below.

Hence, the inventive reversibly PEGylated nanocarrier comprises an inner core comprising or consisting of a nanoparticle and/or at least one pharmaceutical compound. The inner core may be solid or semi-solid/semi-liquid. The inner core may also be hollow or non-hollow.

Such a nanoparticle in the context of the present invention may be any type of particle and preferably defines particles with a spherical or substantially spherical shape typically between 10 and 1000 nm, more preferably with a size of between 10 to 800 nm, more preferably between 10 and 600 nm, even more preferably between 50 and 400 nm.

Such a nanoparticle may be preferably selected from solid or semi-solid/semi-liquid particles. Solid particles may be, for example, selected from or formed by chemical or pharmaceutical compounds, which are provided in a nanoparticulate form. Preferably, the term "pharmaceutical compound" as defined herein is to be understood as a "pharmaceutically active compound". Such a chemical or pharmaceutical compound may be any suitable chemical or pharmaceutical compound as described herein below. Solid particles also may be selected or prepared from compositions of the at least one or more chemical or pharmaceutical compounds, e.g. a mixture of the at least one or more chemical or pharmaceutical compounds, which are preferably provided in a nanoparticulate form. Solid particles may also comprise or be formed by polymers, e.g. mixtures of such chemical or pharmaceutical compounds or compositions thereof with polymers, preferably hydrophilic polymers selected e.g. from PEG, PLA, PLGA, etc. The nanoparticle may furthermore comprise Dextran, e.g. Dextran beads, inert compounds, polymers selected e.g. from PEG, PLA, PLGA, etc. Semisolid/semi-liquid particles may be obtained e.g. from solutions or preferably highly viscous solutions, either with or without such a chemical or pharmaceutical compound as defined herein below, or from solutions, such as e.g. highly viscous solutions, formed e.g. by polymers, preferably hydrophilic polymers selected e.g. from PEG, PLA, PLGA, etc. Nanoparticles may be also selected from vesicles. Likewise, the vesicles may comprise a pharmaceutical compound as described herein below. Preferably, the nanoparticle contained in the inner core is hydrophilic or lipophilic at its outer surface.

The inner core may likewise comprise directly at least one pharmaceutical compound as defined herein below. Typically, the at least one pharmaceutical compound may be provided as a nanoparticle as described before. However, the at least one pharmaceutical compound may also be provided in form of a powder, e.g. a nanopowder, or as a fine or ultrafine powder, or as agglomerates of ultrafine particles, nanoclusters, nanocrystals. Likewise, the at least one pharmaceutical compound may be liquid or semi-liquid/semi-solid, and/or may be formulated as a suspension, e.g. with higher viscosity, so as to form a nanoparticle material. Preferably, the at least one pharmaceutical compound contained in the inner core is hydrophilic or lipophilic.

Exemplary pharmaceutical compounds or pharmaceutically active compounds include, without being limited thereto, e.g. tumoricidal agents such as 2,4,6-triethylene melamine compound, 6-mercaptopurine, ACNU, actinomycin D, adenosinediialde-hgde, adriamycin, AE-941, AG3340, andaminopterin, anti-VEGF antibody, Bay 12-9556, BCNU, bleomycin, BM S-275291, busulfan, CA I, camptothecin, carboplatin, CCNU, Chlorambucil, cisplatin, COL-3, cyclophosphamide, cytarabine, dacarbazine, dasatinib, daunorubicin, doxorubicin, EMD 121974, endostatin, epirubicin, erlotinib, etoposide, floxuridine, fluorouracil, ftorafur, furtulon, gefitinib, guanazole, harringtonine, HXM, hydroxy camptothecin, hydroxyurea, ifosfamide, IM8624, imatinib, indirubin, inosine dialdehyde, interferon [alpha], interleukin 12, irinotecan, lapatinib, liposomal p53 DNA, marimastat, methotrexate, methyl CCNU, mithramycin, mitomycin A, mitomycin B, mitomycin C, mitoxantrone, navelbine, neovastat, nilotinib, nitrogen mustard, N-methyl-N-nitrosourea, olivomycin, oxaliplatin, paclitaxel, pingyangmycin, pirarubicin, PKN3siRNA, procarbazine, sorafenib, squalamine, streptozotocin, SU5416, SU6668, sunitinib, suramin, taxotere, tegadifur, tegafur-uracil, thalidomide, thiotepa, TNP-470, vinblastine, vincristine, vindesine, vinorelbine, vitaxin, etc., gene therapeutic agents, such as DNA, pDNA, RNA, siRNA, miRNA, or a combination thereof, preferably encoding a therapeutic protein, or any further suitable protein, etc.

The nanoparticle and/or at least one pharmaceutical compound forms an inner core of the inventive reversibly PEGylated nanocarrier system. Such an inner core preferably has a spherical or substantially spherical shape, preferably of a size as described above for the nanoparticle, more preferably with a size of between 10 to 1000 nm, more preferably between 10 and 800 nm, even more preferably between 50 and 600 nm or even between 50 and 400 nm. In the context of the inventive reversibly PEGylated nanocarrier system a "spherical or substantially spherical shape" is not necessarily limited to regular globular forms but may also comprise more irregularly shaped forms, ellipsoid formed spheres, etc.

The inner core of the reversibly PEGgylated nanocarrier system may comprise optionally at least one lipid bilayer, the (first) lipid bilayer preferably attached on the surface of the inner core. Such a lipid bilayer is typically prepared by a skilled person following established techniques in the art. A lipid bilayer as preferably used in the context of the present invention is typically formed by phospholipids, cholesterol, and/or glycolipids, wherein phospholipids usually form the basic component of such lipid bilayers. Compounds used in this context include either naturally occurring or synthetic phospholipids, e.g. naturally occurring phospholipids, such as e.g. lecithin or phosphatidylcholin, modified naturally occurring phospholipids, such as e.g. hydrated phosphatidylcholin, semisynthetic phospholipids, such as phospholipids with modified alkyl chains and full synthetic phospholipids.

Preferably, phospholipids, without being limited thereto, may be selected from the group consisting of DSPE (1,2-distearoyl-sn-glycero-3-phosphoethanolamine), PI (phosphatidyl inositol), PS (phosphatidyl serine), DPG (bisphosphatidyl glycerol), PA (phosphatidic acid), PEOH (phosphatidyl alcohol), PG (phosphatidyl glycerol), phosphatidylcholin, hydrogenated soy phosphatidylcholin (HSPC), dimyristoyl phosphatidylcholine (DMPC), etc.

More preferably, without being limited thereto, the phospholipid is a phosphoethanolamine and/or a phosphatidylcholin, most preferably DSPE (1,2-distearoyl-sn-glycero-3-phosphoethanolamine) and/or hydrogenated soy phosphatidylcholin (HSPC). Even more preferably, formation of a lipid bilayer occurs by using cholesterol and a phospholipid as defined before, most preferably cholesterol, DSPE (1,2-distearoyl-sn-glycero-3-phosphoethanolamine) and hydrogenated soy phosphatidylcholin (HSPC), or any derivative thereof, such as modified HSPC, modified cholesterol or modified DSPE, as further explained below.

An optional at least one lipid bilayer as described herein may be a unilamellar or a multilamellar lipid bilayer. Hence, on the inner core of the inventive reversibly PEGylated nanocarrier system an unilamellar or a multilamellar liposome may be formed, preferably selected from MLV, SUV and LUV as further defined below. In case that only one lipid biliayer is defined herein, such one lipid biliayer is preferably arranged on the inner core in form of a unilamellar liposome. In case that more than one lipid biliayer is defined herein, such lipid biliayers are preferably arranged on the inner core in form of a multilamellar liposome. In the inventive context multilamellar liposomes (MLV) in the context of the present invention usually range from 500 to 10,000 nm. Unilamellar liposomes in the context of the present invention can be distinguished into small (SUV) and large (LUV) unilamellar liposomes. SUV are usually smaller than 50 nm and LUV are usually larger than 50 nm. Liposomes of very large size are called giant liposomes (10,000 - 1,000,000 nm), which may be also formed within the context of the present invention. Such giant liposomes can be either unilamellar or multilamellar. Liposomes containing encapsulated vesicles are called multi-vesicular liposomes and are also encompassed by the present invention. Their size typically ranges from 2,000-40,000 nm.

The optional at least one lipid bilayer may be formed on the inner core of the inventive reversibly PEGylated nanocarrier system by any method known to a skilled person. MLV may be prepared e.g. following the hydration method or the solvent spherule method. SUV may be prepared following e.g. the sonication method or the French Pressure method. LUV may be prepared using e.g. either the REV method, the modified REV method, the Freeze Thaw method, the microfluidization method or using a method based on extrusion through polycarbonate filters under nitrogen. Industrial methods for preparing the at least one lipid bilayer include inter alia Detergent Dialysis, Microfluidization, Proliposomes, Lyophilization, etc. Reference with regard to any of these methods is explicitly made in this regard to the publication of RIAZ (1996) (see RIAZ, Pakistan Journal of Pharmaceutical Sciences, Vol.19(1), January 1996, pp.65-77), the respective disclosure of which is entirely incorporated by reference with respect to the type of liposomes and their methods of preparation, particularly as referred to on pages 68 to 75. Other methods suitable for preparing the at least one lipid bilayer are likewise preferred.

According to the present invention, the inventive reversibly PEGylated nanocarrier system comprises a first ligand bound to the surface of said inner core as defined above. Alternatively, if a lipid bilayer is present as defined above, a first ligand may be bound to the surface of said at least one lipid bilayer.

In the context of the present invention, such a first ligand is preferably selected from the following first ligand/second ligand combinations, wherein the first ligand specifically but reversibly binds the second ligand upon addition of the first ligand to the second ligand, or vice versa. In this context, a first ligand/second ligand combination is typically to be understood as a combination of ligands wherein a first ligand, e.g. a protein, such as a restriction enzyme, specifically recognizes and reversibly binds to a second ligand, e.g. its specific DNA recognition sequence. Alternatively, a first ligand, e.g. an enzyme, is specifically bound by a second ligand, e.g. its specific inhibitor. These bonds are reversible, preferably non-covalent, and typically can be released again by competitive binding. Such a reversible attachment of a first ligand to the second ligand thus both allows binding and also competitive binding of a first ligand to a second ligand as defined herein. Hence, according to one aspect of the present invention preferred first ligand/second ligand combinations may be selected from the following combinations, without being limited thereto:

| **First ligand** | **Second ligand** |
|---|---|
| Fluorescein, FITC | scFv fragment FITC-E2 |
| Fluorescein, FITC | scFv fragment FITC-E2 according to SEQ ID NO: 1 or 19 or a sequence showing at least 80% identity to the sequence of SEQ ID NO: 1 or 19 |
| Biotin | anti-Biotin scFv Fragment |

The above defined "first ligands" and the above defined "second ligands" of a first ligand/second ligand combination as defined may also be interchanged, i.e. the first ligand may be used as a second ligand and the second ligand may be used correspondingly as a first ligand.

According to a particularly preferred aspect, the second ligand as defined herein is selected from scFv fragment FITC-E2 and fluorescein compound FITC is selected as a first ligand. More preferably the first ligand/second ligand combination as defined herein is selected from the scFv fragment FITC-E2 according to SEQ ID NO: 1 as a second ligand and FITC as a first ligand. In this context, the scFv fragment FITC-E2 is a single chain variable fragment (scFv) of an antibody directed against fluorescein as published in Vaughan et al. 1996 (see Vaughan et al., (1996), Nat. Biotechnol 14 (3), S. 309-314). Such an scFv may be recombinantly produced as a C-terminal hexahistidine tagged version in established expression systems as described for example by Pedrazzi et al., (1997), FEBS Lett 415 (3), S. 289-293), which shows an expression in the periplasm of *E.coli,* or by Rippmann et al., (1998), Appl. Environ. Microbiol 64 (12), S. 4862-4869), which shows an expression in L-form cells of *Proteus mirabilis.* Expression in either *E. coli* or *Proteus mirabilis* is particularly preferred. A preferable protein sequence of the second ligand may be the amino acid sequence of the produced scFv as shown in SEQ ID NO: 1 or 19 (sequence depicted after removal of the periplasmic signal sequence), or an amino acid sequence showing at least 50, 60, 70, or 80%, preferably at least 90%, more preferably at least 95%, and even more preferably at least 97.5% identity to the sequence according to SEQ ID NO: 1 or 19. The first ligand bound by such a scFv fragment FITC-E2 is fluorescein, particularly FITC, and eventually also a derivative thereof.

As defined before, the first ligand is bound to the surface of said inner core as defined above. Such a binding of the first ligand to the surface of the inner core as defined above occurs typically via covalent or non-covalent bonds, preferably via covalent bonds.

Furthermore, if the inventive reversibly PEGylated nanocarrier system comprises at least one lipid bilayer as defined before, the first ligand is preferably bound to the surface of said at least one lipid bilayer. If a unilamellar lipid bilayer is used, the first ligand is preferably bound to the outer surface of said unilamellar lipid bilayers. If multilamellar lipid bilayers are used, the first ligand is preferably bound to the most outer surface of said multilamellar bilayers, but may be generally also bound to any of the more inner lipid bilayers. Likewise, such a binding of the first ligand to the surface of the at least one lipid bilayer occurs typically via a covalent or non-covalent bond, preferably via a covalent bond. According to a particularly preferred aspect of the present embodiment, the first ligand is bound, preferably covalently bound, to a phospholipid, a cholesterol, and/or a glycolipid or a derivative thereof, as defined above. Such a first ligand, which is preferably covalently bound to a phospholipid, a cholesterol, and/or a glycolipid, can then be integrated into the at least one lipid bilayer when forming same around the inner core of the reversibly PEGylated nanocarrier system as defined herein.

Hence, following a preferred example a first ligand as defined above, is covalently coupled to a phospholipid, a cholesterol, and/or a glycolipid or a derivative thereof, as defined above, wherein such a phospholipid, a cholesterol, and/or a glycolipid or a derivative thereof is preferably used for forming the lipid bilayer structure of the optional at least one lipid bilayer as described above, e.g. unilamellar or multilamellar lipid bilayers structures. Covalent coupling preferably occurs by formation of a chemical bond between the first substrate and the phospholipid, cholesterol, and/or glycolipid or a derivative thereof. A covalent bond between the first ligand and the phospholipid, cholesterol, and/or glycolipid or a derivative thereof may be achieved e.g. by formation of a thiocarbamide adduct or bond between the first ligand and the phospholipid, cholesterol, and/or glycolipid or a derivative thereof, or any further suitable covalent bond known to a skilled person and suitable in the present context.

A particularly preferred example of such an adduct comprises as a first ligand a fluorescein or FITC as defined above, which is preferably coupled via a covalent bond, e.g. by formation of a thiocarbamide adduct, to the phospholipid, cholesterol and/or glycolipid or a derivative thereof. Such a phospholipid, cholesterol and/or glycolipid or a derivative thereof, which is modified by covalently binding a first ligand can then be integrated into the phospholipid bilayer. The formed lipid bilayer then preferably carries on its surface a first ligand as described herein, preferably covalently bound to a member of the lipid bilayer.

More preferably an adduct as described before between the first ligand and the phospholipid, cholesterol, and/or glycolipid or a derivative thereof is provided comprising a fluorescein or FITC as defined above as a first ligand, which is furthermore coupled via a covalent bond, e.g. by formation of a thiocarbamide adduct, to a phosphatidylethanolamine as described above, more preferably to DSPE (1,2-distearoyl-sn-glycero-3-phosphatidylethanolamine). Such an adduct between FITC and DSPE is preferably termed DSPE-FITC.

According to the present invention, the inventive reversibly PEGylated nanocarrier system also comprises a second ligand, which is bound to the first ligand, the second ligand being furthermore covalently bound to at least one biocompatible, soluble and hydrophilic polymer, preferably selected from poly(ethylene glycol) or a derivative thereof.

Preferably, the second ligand is selected in this context from the above defined first ligand/second ligand combinations. More preferably, the second ligand is selected from an scFv fragment FITC-E2, e.g. an scFv fragment FITC-E2 according to SEQ ID NO: 1 as defined above. The corresponding first ligand may be selected from e.g. a fluorescein, preferably FITC. Alternatively, a first ligand may be selected in this context from any compound binding to a scFv fragment FITC-E2 according to SEQ ID NO: 1. The first ligand is optionally modified by covalent coupling to a phospholipid, a cholesterol and/or a glycolipid or a derivative thereof as described before.

The second ligand is covalently bound to at least one biocompatible, soluble and hydrophilic polymer, preferably at least one poly(ethylene glycol) (PEG) or a derivative thereof as defined herein. Preferably, the second ligand is covalently bound to PEG. Covalent binding of PEG or a derivative thereof to the second ligand may occur via free NH₂ groups at the surface of the second ligand. As an example, the single chain antibody fragment FITC-E2 as defined above exhibits 8 lysines and the N-terminus, adding up to 9 potential PEGylation sites, i.e. sites, where PEG could be covalently bound. Binding may also occur via any further suitable groups or moieties of the second ligand, wherein such suitable groups or moieties are known to a skilled person.

PEG as used in the present invention is a biocompatible, soluble and hydrophilic polymer. Herein, the term "poly(ethylene glycol)" or "PEG" is preferably to be understood to be any such biocompatible, soluble and hydrophilic polymer soluble in water and containing ether groups linked by 2 or 3 carbon atoms, optionally branched alkylene groups. The definition of a poly(ethylene glycol) includes branched or non-branched poly(ethylene glycol), poly(propylene glycols), and also block or random copolymers including the two types of units.

Poly(ethylene glycol) as defined herein are water-soluble polymers that have been approved for the oral, parenteral and topical administration of drugs (FDA). Poly(ethylene glycol)s are produced by means of polymerization of ethylene oxide (EO) or propylene oxide (PO) in the presence of water, monoethylene glycol or diethylene glycol as reaction initiators in an alkaline medium ("1,2-Epoxide Polymers: Ethylene Oxide Polymers and Copolymers" in Encyclopedia of Polymer Science and Engineering; Mark, H.F. (Ed.), John Wiley and Sons Inc., 1986, pp. 225-273). When the desired molecular weight (generally controlled by means of in-process measurements of viscosity) is reached, the polymerization reaction is preferably terminated by neutralizing the catalyst with an acid (lactic acid, acetic acid or the like). The result is a linear polymer having a very simple structure: HO-(CH₂-CH₂-O)ₙ-H, wherein (n) is the number of EO monomers or units. The units alternatively contain propylene groups.

PEGylation as described herein also includes PEGylation with derivatives of poly(ethylene glycol) (PEG) as described above, e.g. derivatives of the terminal hydroxyl groups, which can be modified (one or both ends) so as to introduce, without being limited thereto, alkoxy, acrylate, methacrylate, alkyl, amino, phosphate, isothiocyanate, sulfhydryl, mercapto and sulfate groups. The polyethylene glycol or polypropylene glycol can have substituents in the alkylene groups. If they are present, these substituents are preferably alkyl groups.

For the purposes of the present invention and in accordance with common definitions, the term PEG is preferably used in combination with a numerical value. In the pharmaceutical industry the numerical value typically indicates the mean molecular weight. Typically, PEGs with a molecular weight of less than 400 Da are non-volatile liquids at room temperature. PEG 600 shows a melting point between 17 and 22 °C, whereas PEGs with mean molecular weights between 800 and 2000 Da are pasty materials with low melting points. Above a molecular weight exceeding 3000 Da, PEGs are solid and commercially available up to PEG 35000. On the other hand, although the melting point of PEGs increases when the molecular weight increases, the boiling point increases up to a maximum value of 60°C. Likewise, when the molecular weight increases, its aqueous solubility decreases. In any case for PEG 35000, an amount close to 50% w/w can be dissolved in water.

PEG derivatives as described and used herein have advantages that are similar to traditional PEGs such as their aqueous solubility, physiological inactivity, low toxicity and stability under very different conditions. These PEG derivatives include very different products and are characterized by the functional group substituting the hydroxyl, such as -NH₂ (among the most reactive ones), phenol, aldehyde, isothiocyanate, -SH groups, etc. The following PEG derivatives are exemplarily listed as PEG derivatives suitable and provided for use within the context of the present invention, without being limited thereto:
polyoxyethylene esters: PEG monomethyl ether monosuccinimidyl succinate ester; PEG monomethyl ether monocarboxymethyl ether; PEG adipate; PEG distearate; PEG monostearate; PEG hydroxystearate; PEG dilaurate; PEG dioleate, PEG monooleate, PEG monoricinoleate; PEG coconut oil esters.

Polyoxyethylene alkyl ethers: PEG monomethyl ether or methoxy PEG (mPEG); PEG dimethyl ether.

Others: Poly(ethylene glycol terephthalate); polyoxyethylene derivatives and sorbitan esters and fatty acids; ethylene oxide and propylene oxide copolymers; ethylene oxide with acrylamide copolymers.

PEG derivatives: O,O'-Bis-(2-aminoethyl)polyethylene glycol (DAE-PEG 2000); O,O'-Bis-(2-aminopropyl)polypropylene glycol-polyethylene glycol-polypropylene glycol (DAP-PEG 2000).

Following another preferred aspect of the first embodiment a poly(ethylene glycol) derivative may be selected, without being limited thereto, from poly(ethylene glycol) methyl ether, preferably poly(ethylene glycol) methyl ether 2000 (mPEG 2000).

Following another preferred aspect of this embodiment of the invention the poly(ethylene glycol) used has terminal functional groups different from the hydroxyl group, such as amino groups. These amino groups can in turn be substituted and have functional groups. Hence, these amino groups can be used to further modify the poly(ethylene glycol). In a preferred variant the amino groups are -NH₂. It has been observed that with these groups, the oral administration of the nanoparticles accumulate on certain segments of the intestinal tract, which allows a specific administration.

According to an even further preferred aspect, the polyethylene glycol used according to the present invention has amino groups and branches in the alkyl group. It has been found that with these substituents it is possible to form a brush-type structure, with one of the ends inside and the other one on the outside. The chemical structures of some suitable polyalkylene glycols corresponding to the previously mentioned groups with different types of functional groups are, without being limited thereto, illustratively selected from:
a) H(OCH₂CH₂)ₙOH
b) H₃C(OCH₂CH₂)ₙOH
c) H₂N(CH₂CH₂O)ₙCH₂CH₂NH₂
d) H₂NCHCH₃CH₂(OCHCH₃CH₂)(OCH₂CH₂)ₙ(OCH₂CHCH₃)NH₂
wherein n is preferably between 200 to 6000, preferably e.g. 400, 1000, 2000, etc.

Particularly preferred examples of polyalkylene glycols and poly(ethylene glycol) include, without being limited thereto:
a) polyethylene glycol 1000 or 2000 (PEG 1000 or PEG 2000);
b) polyethylene glycol methyl ether 2000 (mPEG 2000);
c) O,O'-Bis-(2-aminoethyl)polyethylene glycol 2000 (DAE-PEG 2000); and
d) O,O'-Bis-(2-aminopropyl)polypropylene glycol- polyethylene glycol-polypropylene glycol (DAP-PEG 2000).

Following a preferred aspect of the first embodiment a poly(ethylene glycol) or a derivative thereof may be selected from e.g. PEG-PE (polyethyleneglycol-phosphatidylethanolamine),PLGA-PEG (block-copolymer of PEG and polylactide-glycolide), etc.

Following a preferred aspect of the first embodiment, a poly(ethylene glycol) or a derivative thereof as used herein is not branched and does not have substituted hydroxyl groups. In this aspect, the poly(ethylene glycol) or a derivative thereof as used herein preferably has a molecular weight between 400 and 35,000 Da. When the molecular weight is less than 400 Da it has been found that PEGylation typically does not efficiently occur. Therefore, in one preferred aspect of the first embodiment, the polyethylene glycol used in manufacturing inventive PEGylated nanocarriers has a molecular weight equal to or greater than 400 Da, more preferably equal to or greater than 1,000 Da, values between 1,500 Da and 10,000 Da are especially preferred, preferably between 500 Da and 10,000 Da, e.g. between 1,000 Da and 10,000 Da, between 1,000 Da and 8,000 Da or between 1,000 Da and 5,000 Da, e.g. 1,000 Da, 2,000 Da, 3,000 Da, 4,000 Da, or 5,000 Da. According to a particularly preferred aspect, poly(ethylene glycol) 2000 (PEG 2000) is used. Any range formed of one of the aforementioned values or ranges is also included, including combinations of the upper and/or lower ranges with any of the single values.

According to the present invention, the second ligand of the reversibly PEGylated nanocarrier system preferably binds to the first ligand and binding of the second ligand to the first ligand is reversible.

Binding of the second ligand to the first ligand typically occurs by adding the second ligand being covalently bound to at least one PEG moiety as described above, usually under reaction conditions well known for a skilled person from the corresponding first ligand/second ligand combinations as described herein.

The binding of the second ligand to the first ligand and correspondingly the PEGylation of the nanocarrier is reversible. Such a detachment of the second linker from the first linker typically occurs via competitive binding, which is induced or caused by addition of an excess amount of the (non-modified) first ligand or second ligand to the reversibly PEGgylated nanocarrier system or by addition of any further compound capable of competitively binding to either the first or the second ligand. Such an addition of an excess amount of the first ligand or second ligand or any further suitable compound as mentioned above releases the nanocarrier system from its PEG-shell/PEGylation by detaching the second ligand from the inventive nanocarrier system. During competitive binding, the detached second ligand with its covalently bound PEG-component binds to the added first ligand and thereby blocks the "binding site" of the detached second ligand for any further interaction with the first ligand of the nanocarrier system. Alternatively, of course, during competitive binding a second ligand may be added, which is not bound to a PEG or a derivative thereof, and then may bind competitively to the first ligand yet bound to the inventive nanocarrier system and thereby detaching the second ligand bound to PEG or a derivative thereof from its binding partner and the inventive nanocarrier system. In any case, detachment of the PEG-shell/PEGylation from the inventive the nanocarrier system occurs. The released inventive nanocarrier system then can further be degraded, preferably in the tumor cell and/or the contained cargo (inner core) may be released.

A (free) first ligand suitable in this context to trigger competitive binding and thereby release of the second ligand with the PEG-component is typically any first ligand as described before in the context of the first ligand/second ligand combinations as outlined herein before or any other further compound capable of competitively binding to the second ligand. Preferably, such a suitable first ligand is a non-modified first ligand from said first ligand/second ligand combinations. A (non-modified) first ligand in the context of the present invention is preferably a first ligand as defined herein, wherein said first ligand is typically not bound, neither covalently nor non-covalently or via any further binding forces to any further compound, particularly preferably not to a member of the at least one lipid bilayer as defined above, i.e. a phospholipid, cholesterol, and/or a glycolipid.

Likewise, a second ligand suitable in this context to trigger competitive binding and thereby release of the second ligand with the PEG-component is typically any second ligand as described before in the context of the first ligand/second ligand combinations or any other further compound capable of competitively binding to the first ligand. Preferably, such a suitable second ligand is a non-modified protein or substrate from said protein/substrate combinations. A (non-modified) second ligand in the context of the present invention is preferably a second ligand as defined herein, wherein said second ligand is typically not bound, neither covalently nor non-covalently or via any further binding forces to any further compound, particularly preferably not to PEG or a derivative thereof as described herein.

The amounts required for competitive binding and hence detachment of the PEGylation from the herein described inventive PEGylated nanocarrier system are typically to be determined on basis of the patient to be treated and usually defined in amount/kg bodyweight of the patient. Such amounts can be readily determined by the skilled person on basis of clinical routine assessments.

According to a further aspect, the inventive reversibly PEGylated nanocarrier system as described herein is modified to allow a specific targeting of the inventive reversibly PEGylated nanocarrier system to a specific location, such as a specific cell, specific tissue, etc.

Such a specific targeting is typically affected by coupling a further third ligand either to the surface reversibly PEGylated nanocarrier system, for the purposes of the present invention termed "third ligand" or to any further component of the inventive reversibly PEGylated nanocarrier system as described herein. Such a third ligand may be any compound selected from an antibody, a protein, a peptide, a small molecule, a sugar, or any further compound, preferably as described in the table below, which is capable to target the inventive reversibly PEGylated nanocarrier system to a specific target region. Exemplary types of "third" ligands may be selected e.g. from the following: anti-HER2, anti-CD9, nucleosome-specific 2C5 mAb, transferrin, interleukin 13 (IL-13), Octreotide, LHRH-derived peptide, Arg-Gly-Asp (RGD), folate, estrone, anisamide, mannos, and/or lactose, etc. The target regions of such a third ligand are preferably selected, without being limited thereto, as depicted in the following table:

**Table 1**

| **Type of ligand** | **Ligand** | **Target** |
|---|---|---|
| Antibody | anti-HER2, | HER2 receptor overexpressed by cancer cells, |
| | anti-CD9, | CD19 overexpressed in B cell lymphoma, |
| | nucleosome-specific 2C5 mAb, | Cancer cells surface-bound nucleosomes, |
| Protein | transferrin, | Transferrin receptor overexpressed by cancer cells, |
| | interleukin 13 (IL-13), | IL-13 receptor overexpressed in human gliomas |
| Peptide | Octreotide, | Somatostatin receptor type 2 overexpressed by cancer cells, |
| | LHRH-derived peptide, | LHRH receptors overabundant on cancer cells, |
| | Arg-Gly-Asp (RGD), | aVb3 overexpressed by endothelial tumor cells, |
| Small molecule | folate, | Folate receptor on cancer cells, |
| | estrone, | Estrogen receptors overexpressed in ovarian and breast cancer, |
| | anisamide, | Sigma receptors overexpressed by cancer cells |
| Sugar | mannose, | Dendritic cells and macrophages to induce an immune response, |
| | lactose, | Asialoglycoprotein receptors overexpressed by hepatocellular carcinomas; |

Third ligands in the context of the present invention for specifically targeting the inventive reversibly PEGylated nanocarrier system to a desired target region may be bound to the inventive reversibly PEGylated nanocarrier system in a non-covalent fashion, e.g. through electrostatic interactions, van-der-Waals interactions, etc., or any further interactions to be subsumed as a non-covalent bond, or via a covalent bond, more preferably via a covalent bond. Such a covalent bond is preferably a covalent bond as defined herein and may be formed accordingly as described herein.

The finally prepared reversibly PEGylated nanocarrier system of the present invention typically has a spherical form. The PEGylated nanocarrier system preferably has a size of about 10 to 1,500 nm, preferably 10 to 1,200 nm, likewise preferably 10 to 1000 nm, 10 to 800 nm, 10 to 600 nm or 10 to 400 nm even more preferably 20 to 300 nm, e.g. 50 to 250 nm or 50 to 200 nm.

According to a further embodiment, the present invention provides a pharmaceutical composition or medicament comprising the inventive reversibly PEGylated nanocarrier system as described herein. The inventive pharmaceutical composition preferably comprises as an active ingredient the reversibly PEGylated nanocarrier system as described herein and optionally a pharmaceutically acceptable carrier. In this context preferred is the use of such a herein described reversibly PEGylated nanocarrier system as a medicament or as a pharmaceutical composition. Likewise preferred is the use of a herein described reversibly PEGylated nanocarrier system in the treatment of any disease as described herein, preferably for the treatment of cancer or in the field of gene therapy. Hence, one aspect of the present embodiment is directed to a herein described nanocarrier system for use in cancer therapy, in gene therapy, in inflammatory diseases or in pain therapy/treatment, without being limited thereto.

In the context of the present invention the term "cancer", e.g. in "cancer therapy", may cover, without being limited thereto a range of possible cancers, such as e.g. Kaposi's sarcoma, any cancer or tumor disease selected from melanomas, malignant melanomas, colon carcinomas, lymphomas, sarcomas, blastomas, renal carcinomas, gastrointestinal tumors, gliomas, prostate tumors, bladder cancer, rectal tumors, stomach cancer, oesophageal cancer, pancreatic cancer, liver cancer, mammary carcinomas (= breast cancer), uterine cancer, cervical cancer, acute myeloid leukaemia (AML), acute lymphoid leukaemia (ALL), chronic myeloid leukaemia (CML), chronic lymphocytic leukaemia (CLL), hepatomas, various virus-induced tumors such as, for example, papilloma virus-induced carcinomas (e.g. cervical carcinoma = cervical cancer), adenocarcinomas, herpes virus-induced tumors (e.g. Burkitt's lymphoma, EBV-induced B-cell lymphoma), hepatitis B-induced tumors (hepatocellularcarcinomas), HTLV-1 - and HTLV-2-induced lymphomas, acoustic neuroma, lung carcinomas (= lung cancer = bronchial carcinoma), small-cell lung carcinomas, pharyngeal cancer, anal carcinoma, glioblastoma, rectal carcinoma, astrocytoma, brain tumors, retinoblastoma, basalioma, brain metastases, medulloblastomas, vaginal cancer, pancreatic cancer, testicular cancer, Hodgkin's syndrome, meningiomas, Schneeberger disease, hypophysis tumor, Mycosis fungoides, carcinoids, neurinoma, spinalioma, Burkitt's lymphoma, laryngeal cancer, renal cancer, thymoma, corpus carcinoma, bone cancer, non-Hodgkin's lymphomas, urethral cancer, CUP syndrome, head/neck tumors, oligodendroglioma, vulval cancer, intestinal cancer, colon carcinoma, oesophageal carcinoma (= oesophageal cancer), wart involvement, tumors of the small intestine, craniopharyngeomas, ovarian carcinoma, genital tumors, ovarian cancer (= ovarian carcinoma), pancreatic carcinoma (= pancreatic cancer), endometrial carcinoma, liver metastases, penile cancer, tongue cancer, gall bladder cancer, leukemia, plasmocytoma, lid tumor, prostate cancer (= prostate tumors), etc.

Additionally, the term "gene therapy" in the context of the present invention likewise concerns genetic diseases, also termed (hereditary) diseases or monogenetic diseases. Such (mono-)genetic diseases, (hereditary) diseases, or genetic diseases in general are typically caused by genetic defects, e.g. due to gene mutations resulting in loss of protein activity or regulatory mutations which do not allow transcription or translation of the protein. Frequently, these diseases lead to metabolic disorders or other symptoms, e.g. muscle dystrophy. Particularly, such (mono-)genetic diseases *inter alia* include Alzheimer disease, Breast cancer-1 (early onset), Breast cancer-2 (early onset), Creutzfeldt-Jakob disease , Diabetes mellitus (insulin-resistant); Diabetes mellitus (rare form); Diabetes mellitus (type II), Mucoviscidosis, Obesity, Osteoarthrosis, Pancreatic cancer, Parkinson disease (familial or juvenile), Sickle cell anemia, etc, without being limited thereto.

The term "inflammatory diseases" in the context of the present invention likewise includes Pelvic Inflammatory Disease (PID) and Symptoms of Abdominal Pain, Fever and Painful Intercourse; gout, which causes pain, soreness, swelling, numbness and reddening in joints e.g. in the big toe; lupus, including systemic lupus erythematosus, which involves many organs such as heart, lungs, kidneys, brain; discoid lupus erythematosus, which mainly affects the skin with a red rash or pigment change of face, scalp; and drug-induced lupus, which is triggered by a few medicines, such as heart condition drugs, more common in men; and also inflammatory diseases such as asthma, pleurisy, eczema, arthritis, gastritis, splenitis, sinusitis, hepatitis, nephritis, psoriasis, vasculitis, laryngitis, thyroiditis, prostatitis, pharyngitis, sarcoidosis, atherosclerosis, allergic reactions, multiple sclerosis, some myopathies, rheumatoid arthritis, seborrheic dermatitis, Wegener's granulomatosis, irritable bowel syndrome (IBS), Crohn's disease, ulcerative colitis, diverticulitis, inflammatory bowel disease (IBD), etc.

The inventive pharmaceutical composition as described above typically comprises the inventive reversibly PEGylated nanocarrier system as described herein and optionally a pharmaceutically acceptable carrier and/or vehicle. In the context of the present invention, a pharmaceutically acceptable carrier typically includes the liquid or non-liquid basis of the pharmaceutical composition. Since the pharmaceutical composition is typically provided in liquid or at least semi-liquid/semi-solid form, the optional carrier will typically be pyrogen-free water; isotonic saline or buffered (aqueous) solutions, e.g. phosphate, citrate etc. buffered solutions. Alternatively, no such carrier may be used. The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used which do not lead to damage of cells due to osmosis or other concentration effects. Reference media are e.g. liquids occurring in *in vivo* methods, such as blood, lymph, cytosolic liquids, or other body liquids, or e.g. liquids, which may be used as reference media in *in vitro* methods, such as common buffers or liquids. Such common buffers or liquids are known to a skilled person.

However, one or more compatible liquid fillers or diluents or encapsulating compounds may be used as well for the pharmaceutical composition, which are suitable for administration to a patient to be treated. The term "compatible" as used here means that these constituents of the pharmaceutical composition are capable of being mixed with the reversibly PEGylated nanocarrier system as defined herein in such a manner that no interaction occurs which would substantially reduce the pharmaceutical effectiveness of the pharmaceutical composition and its active ingredients under typical use conditions. Pharmaceutically acceptable carriers, fillers and diluents must, of course, have sufficiently high purity and sufficiently low toxicity to make them suitable for administration to a person to be treated. Some examples of compounds which can be used as pharmaceutically acceptable carriers, fillers or constituents thereof are sugars, such as, for example, without being limited thereto, lactose, glucose and sucrose; starches, such as, for example, corn starch or potato starch; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulfate; vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid.

The inventive pharmaceutical composition may be administered, without being limited thereto, orally, parenterally, topically, rectally, nasally, buccally, vaginally or via an implanted reservoir. The term parenteral as used herein includes subcutaneous, intravenous, intramuscular, intra-articular, intra-nodal, intra-synovial, intrasternal, intrathecal, intrahepatic, intralesional, intracranial, transdermal, intradermal, intrapulmonal, intraperitoneal, intracardial, intraarterial, and sublingual injection or infusion techniques. Preferably, the inventive pharmaceutical composition may be administered by parenteral injection, more preferably by subcutaneous, intravenous, intramuscular, intra-articular, intra-nodal, intra-synovial, intrasternal, intrathecal, intrahepatic, intralesional, intracranial, transdermal, intradermal, intrapulmonal, intraperitoneal, intracardial, intraarterial, and sublingual injection or via infusion techniques. Particularly preferred is intradermal, subcutaneous and intramuscular injection. Sterile injectable forms of the pharmaceutical compositions may be aqueous or oleaginous suspension. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di- glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation of the pharmaceutical composition.

The inventive pharmaceutical composition as defined herein may also be administered orally in any orally acceptable dosage form including, but not limited to, capsules, aqueous suspensions, gels or solutions. For oral administration in a capsule form, useful carriers include lactose and dried cornstarch. When aqueous suspensions are required for oral use, the active ingredient, i.e. the complex, is combined with emulsifying and suspending agents. If desired, certain sweetening, flavoring or coloring agents may also be added.

The inventive pharmaceutical composition typically comprises a "safe and effective amount" of the active ingredients of the pharmaceutical composition, particularly of the reversibly PEGylated nanocarrier system as described herein. As used herein, a "safe and effective amount" means an amount of the active ingredients sufficient to significantly induce a positive modification of a disease or disorder as defined herein. At the same time, however, a "safe and effective amount" is small enough to avoid serious side-effects and to permit a sensible relationship between advantage and risk. The determination of these limits typically lies within the scope of sensible medical judgment. A "safe and effective amount" of the active ingredient of the inventive pharmaceutical composition will furthermore vary in connection with the particular condition to be treated and also with the age and physical condition of the patient to be treated, the body weight, general health, sex, diet, time of administration, rate of excretion, drug combination, the activity of the complex or of the antigen, the severity of the condition, the duration of the treatment, the nature of the accompanying therapy, of the particular pharmaceutically acceptable carrier used, and similar factors, within the knowledge and experience of the accompanying doctor. The pharmaceutical composition may be used for human and also for veterinary medical purposes, preferably for human medical purposes, as a pharmaceutical composition in general or as a vaccine.

As used herein, the singular form of a word also includes the plural and vice versa unless the context clearly dictates otherwise. Thus, the terms "a", "an" and "the" are generally meant to include the singular and the plurals of said terms. Similarly, the terms "comprise, "comprises" and "comprising" are to be interpreted inclusively rather than exclusively. Likewise, the terms "examples", "e.g.", and "such as", particularly when followed by a listing of terms is merely exemplary and illustrative and should not be deemed to be exclusive or comprehensive. Unless defined otherwise, all technical scientific terms, terms of the art and acronyms as used herein have the meanings commonly understood by one of ordinary skill in the art in the field.

Any references cited or referred to herein are entirely incorporated herein by reference to the extent allowed by law. The discussion of those references is intended to summarize the assertions made therein. No admission is made that any such references or any portion thereof are relevant material or prior art. The right to challenge the accuracy and pertinence of any assertion of such references as relevant material or prior art is specifically reserved.

Having thus described the present invention and the advantages thereof, it should be appreciated that the various aspects and embodiments of the present invention as disclosed herein are merely illustrative of specific ways to make and use the invention.

### Examples

The following Examples shall illustrate the above described invention in further detail and are not intended to limit the scope of the claims thereto.

### Example 1 Exemplary setup of the reversible PEGylation nanocarrier system - liposomal nanocarrier

### a) Reference Liposome preparation (Doxorubicin (Doxil®-like liposomes))

As a reference system, a liposomal formulation of Doxorubicin (Doxil®-like liposomes) was prepared following common protocols using HSPC, Cholesterol and DSPE-mPEG in the following amounts:

| | |
|---|---|
| Hydrogenated soy phosphatidylcholin (HSPC): | 9.58 mg/ml |
| Cholesterol (Chol): | 3.19 mg/ml |
| N-(carbonylmethoxypolyethyleneglycol 2000)-1,2-distearoyl-sn-glycerol-3-phosphoethanolamine sodium salt (DSPE-mPEG) (see also www.doxil.com/assets/DOXIL_PI_Booklet.pdf) | 3.19 mg/ml |

The Doxil®-like liposome preparation was prepared using the following protocol:
The liposomes were produced by the film-hydration method. The lipids were dissolved in chloroform to a final concentration of 16 mg/ml, followed by evaporation under vacuum. The resulting lipid film is afterwards rehydrated in HEPES-buffer pH = 7.4 in a water bath at 65°C. The resulting polydisperse liposome-solution is further downsized by extrusion through a polycarbonate membrane with a pore size of 80 nm. Size and dispersion were measured by dynamic light scattering to ensure uniformity of the liposomes.

(For further details of preparation: "Liposomes: A Practical Approach"; Vladimir P. Torchilin and Volkmar Weissig)

### b) Inventive Liposome preparation (Doxorubicin (Doxil®-like liposomes))

### b1) Preparation of DSPE-FITC adduct

For the inventive purposes, the PEGylated DSPE as used in Example 1a) above was replaced by DSPE coupled to fluorescein. The coupling was carried out as a thiocarbamide adduct. The resulting compound has been termed DSPE-FITC. The reaction is exemplarily depicted in the following.

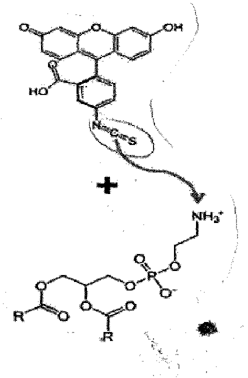

The compound DSPE-FITC has been then used to prepare a liposomal formulation of Doxorubicin (Doxil®-like liposomes) was prepared following common protocols using in a first step HSPC, Cholesterol and DSPE-FITC in the following amounts: In a second step FITC-E2 was coupled to DSPE-FITC and PEGylating the FITC-E2 via free NH₂-groups.

### b2) Preparation of liposomal formulation of Doxorubicin (Doxil®-like liposomes) with FITC-E2/ DSPE-FITC

Due to the different molecular weights of DSPE-mPEG (2805 g/mol) and DSPE-FITC (1166 g/mol), the final composition was changed to:

| | |
|---|---|
| Hydrogenated soy phosphatidylcholin (HSPC): | 9.58 mg/ml |
| Cholesterol (Chol): | 3.19 mg/ml |
| DSPE-FITC: | 1.32 mg/ml |

The scFv used in the inventive example, FITC-E2 (see Pedrazzi G, Schwesinger F, Honegger A, Krebber C, Plückthun A (1997) FEBS Lett, 415(3): 289-93), binds fluorescein with high specificity and has a molecular weight of ca. 28 kDa. The PEGylation of the protein was successfully achieved by reaction of NHS-mPEG2000 at free -NH₂ groups (see above). FITC-E2 has 8 lysines and the N-terminus, adding up to 9 potential PEGylation sites.

PEGylation of the FITC-E2 was visualized on a 12% SDS PAGE (see Figure 2). MM represents the molecular marker. scFv represents FITC-E2, scFv+PEG represents the single short chain fragment FITC-E2 modified with PEG (PEGylated; In lane "scFV" 20 µl FITC-E2 (18mg/ml) were loaded in the corresponding lane. In the lane "scFv+PEG" scFv was reacted with 6x excess of NHS-mPEG2000 with the same loading conditions as for scFv.

### Example 2 Test of the functionality of the inventive reversibly PEGylated nanocarrier system on THP-1 cells

a) The functionality of the setup was tested in a macrophage uptake assay using THP-1 cells (Human acute monocytic leukaemia cell were differentiated prior to the experiments to macrophage-like cells. This assay simulates the reaction of the immune system to an injection of the liposomes into the human body (see Blume G, Cevc G (1990) Biochim Biophys Acta, 1029(1):91-7).
   Prior to the test, the liposomes were stained with DiD (Vybrant® DiD Cell-Labeling Solution). Then, the THP-1 cells were incubated 90 minutes with different formulations of liposomes (liposomes, liposomes + scFv-PEG or liposomes + scFv-PEG + fluorescein) or just buffer (HEPES). Cells were washed three times with PBS 37 °C and then detached with PBS + 2 mM EDTA. Relative fluorescence of THP-1 cells was afterwards determined with a flow cytometer.
   As could be seen in Figure 3, the fluorescence of the cells correlates with the uptake of DiD stained liposomes by the macrophages. The determined uptake rate is comparable with the figures obtained with conventionally PEGylated liposomes (see Blume G, Cevc G (1990) Biochim Biophys Acta, 1029(1):91-7), and is nearly 100% reversible after addition of fluorescein to a final concentration of 1 mM.
   Figure 4, as outlined above, shows fluorescein-dependent scFv-PEG de-shielding of the nanocarrier (liposome) in the presence of THP-1 cells. For this purpose, THP-1 cells were incubated 90 minutes with bare liposomes or liposomes with scFv-PEG and different concentrations of fluorescein (500 µM, 250 µM, 125 µM, 60 µM, 30 µM, 15 µM and just HEPES-buffer). Cells were washed three times with PBS 37 °C and then lysed with HEPES 2% Triton-X. The uptake was quantified by measuring the DiD fluorescence, which the liposomal membranes were stained with (L: liposomes; F: fluorescein).
   In Figure 5 further results can be seen. Figure 5 shows a test of different concentration of scFv-PEG to provide the scFv-PEG shell protection to the nanocarrier (liposome) in the presence of THP-1 cells. THP-1 cells were incubated 90 minutes with bare liposomes or liposomes with different scFv-PEG concentrations (5mol%-PE-FITC in liposomal membrane; molar ratio scFv-PEG:PE-FITC: 1:2; 1:1; 2:1, 4:1, or BSA 4:1). Cells were washed three times with PBS 37 °C and then lysed with HEPES 2% Triton-X. The uptake was quantified by measuring the DiD fluorescence, which the liposomal membranes were stained with (L: liposomes).
b) Furthermore, a test of the functionality of the inventive reversibly PEGylated nanocarrier system in a CHO-cell proliferation assay has been carried out. For this purpose, CHO cells were incubated for 4 hours with doxorubicin or doxorubicin loaded liposomes in combination with the protective shell with or without the addition of fluorescein (HEPES, Doxorubicin, Doxorubicin liposomes + scFv-PEG or doxorubicin liposomes + scFv-PEG + fluorescein). Cells were washed three times with PBS 37 °C and then further cultivated for 24 h. To quantify the cell proliferation a WST-1 assay (Roche) was performed. Results are presented in Figure 6.
c) In addition, the plasma half-life of Doxorubicin loaded into liposomes with PEGylated scFv was tested in *Rattus norvegicus* (Dosage 3.75 mg/rat). A prolonged half-life (19.5 h) of the protected liposomal doxorubicin can be seen compared to literature data for free doxorubicin (<1h) or liposomal, unprotected, doxorubicin (1-2h). Results are presented in Figure 7.

### Example 3 Exemplary setup of the reversible PEGylation nanocarrier system - non-liposomal nanocarrier

### a) Protocol for production of FITC modified dextran nanoparticles:

Amino-modified dextran-nanoparticles are dissolved in 50% EtOH with 100 mM sodium bicarbonate. A 2-times molar excess of FITC in relation to amino-groups is dissolved in EtOH and an equimolar amount of triethanolamine is added. Afterwards, the amino dextran particles are mixed with the FITC-solution and incubated for at least 4 hours at 4°C. The unreacted FITC is afterwards removed by dialysis (Cutoff 3,5 kDa) against PBS pH = 7.4.

### Example 4 Exemplary setup of the reversible PEGylation nanocarrier system based on biotin -scFv interaction

### Preparation of DSPE-biotin adduct

For the inventive purposes, the PEGylated DSPE as used in Example 1a) above was replaced by DSPE coupled to biotin. The coupling was carried out as N-hydroxysuccinimido adduct with the free amino group of the phosphatidylethanolamine. The resulting compound has been termed DSPE-biotin. The compound DSPE-biotin has been then used to prepare a liposomal formulation following common protocols using in a first step HSPC, Cholesterol and DSPE-Biotin in the above mentioned amounts. In a second step previous -NH₂ PEGylated anti-biotin scFv was coupled to DSPE-biotin.

## Claims

1. Reversibly PEGylated nanocarrier system comprising:
- an inner core comprising a nanoparticle and/or at least one pharmaceutical compound;
- a first ligand bound to the surface of said inner core;
- a second ligand bound to the first ligand, the second ligand being furthermore covalently bound to poly(ethylene glycol) or a derivative thereof,
**characterized in that** binding of the second ligand to the first ligand is non-covalent and is reversible, wherein the first ligand and the second ligand are selected from the following first ligand/second ligand combinations:
| **First ligand** | **Second ligand** |
|---|---|
| Fluorescein, FITC | scFv fragment FITC-E2 |
| Fluorescein, FITC | scFv fragment FITC-E2 according to SEQ ID NO: 1 or 19 or a sequence showing at least 80% identity to the sequence of SEQ ID NO: 1 or 19 |
| Biotin | anti-Biotin scFv Fragment |

2. Reversibly PEGylated nanocarrier system according to claim 1, wherein the nanocarrier system further comprises at least one lipid bilayer on the surface of the inner core, wherein the first ligand is then bound to the surface of at least one lipid bilayer.

3. Reversibly PEGylated nanocarrier system according to claim 2, wherein the nanocarrier system has a mono- or a multilamellar lipid bilayer on the surface of the inner core, wherein the first ligand is bound to the surface of said at least one lipid bilayer.

4. Reversibly PEGylated nanocarrier according to any of claims 1 to 3, wherein the second ligand is covalently bound to poly(ethylene glycol) or a derivative thereof.

5. Reversibly PEGylated nanocarrier system according to any of claims 1 to 4, wherein the molecular weight of the poly(ethylene glycol) or a derivative thereof is in the range between 500 and 10,000 gmol⁻¹, preferably between 1,000 and 8,000 gmol⁻¹, more preferably between 1,000 and 5,000 gmol⁻¹.

6. Reversibly PEGylated nanocarrier system according to any of claims 1 to 5, wherein the nanocarrier system has a size of about 10 to 1,500 nm, preferably 10 to 1,200 nm, likewise preferably 10 to 1,000 nm, 10 to 800 nm, 10 to 600 nm or 10 to 400 nm even more preferably 20 to 300 nm, 50 to 250 nm or 50 to 200 nm.

7. Reversibly PEGylated nanocarrier system according to any of claims 1 to 6, wherein the at least one pharmaceutical compound is a tumoricidal agent selected from the group consisting of 2,4,6-triethylene melamine compound, 6-mercaptopurine, ACNU, actinomycin D, adenosinediialde-hgde, adriamycin, AE-941, AG3340, andaminopterin, anti-VEGF antibody, Bay 12-9556, BCNU, bleomycin, BM S-275291, busulfan, CA I, camptothecin, carboplatin, CCNU, Chlorambucil, cisplatin, COL-3, cyclophosphamide, cytarabine, dacarbazine, dasatinib, daunorubicin, doxorubicin, EMD 121974, endostatin, epirubicin, erlotinib, etoposide, floxuridine, fluorouracil, ftorafur, furtulon, gefitinib, guanazole, harringtonine, HXM, hydroxy camptothecin, hydroxyurea, ifosfamide, IM8624, imatinib, indirubin, inosine dialdehyde, interferon [alpha], interleukin 12, irinotecan, lapatinib, liposomal p53 DNA, marimastat, methotrexate, methyl CCNU, mithramycin, mitomycin A, mitomycin B, mitomycin C, mitoxantrone, navelbine, neovastat, nilotinib, nitrogen mustard, N-methyl-N-nitrosourea, olivomycin, oxaliplatin, paclitaxel, pingyangmycin, pirarubicin, PKN3siRNA, procarbazine, sorafenib, squalamine, streptozotocin, SU5416, SU6668, sunitinib, suramin, taxotere, tegadifur, tegafur-uracil, thalidomide, thiotepa, TNP-470, vinblastine, vincristine, vindesine, vinorelbine, and/or vitaxin, or a combination thereof, and/or the at least one pharmaceutical compound is a gene therapeutic agent.

8. Reversibly PEGylated nanocarrier system according to any of claims 1 to 7, wherein the at least one pharmaceutical compound comprises or is selected from DNA, RNA, pDNA or siRNA, or wherein the nanoparticle comprises such a pharmaceutical compound.

9. Reversibly PEGylated nanocarrier system according to any of claims 1 to 8, wherein the nanoparticle comprises dextran or a polymer selected from PEG, PLA, PLGA, or an inert compound, or may be a vesicle.

10. Reversibly PEGylated nanocarrier system according to any of claims 1 to 9, wherein the nanocarrier system comprises a third ligand attached to the nanocarrier system for specifically targeting the reversibly PEGylated nanocarrier system to a desired target region.

11. Reversibly PEGylated nanocarrier system according to claim 10, wherein said third ligand is selected from anti-HER2, anti-CD9, nucleosome-specific 2C5 mAb, transferrin, interleukin 13 (IL-13), Octreotide, LHRH-derived peptide, Arg-Gly-Asp (RGD), folate, estrone, anisamide, mannose, and/or lactose.

12. Pharmaceutical composition comprising the reversibly PEGylated nanocarrier system according to any of claims 1 to 11 and optionally a pharmaceutically acceptable carrier and/or vehicle.

13. Reversibly PEGylated nanocarrier system according to any of claims 1 to 11 for use as a medicament.

14. Reversibly PEGylated nanocarrier system according to any of claims 1 to 11 or a pharmaceutical composition according to claim 12 for use in cancer therapy, gene therapy, pain therapy or the treatment of inflammatory diseases.

## Patentansprüche

1. Reversibel PEGyliertes Nanocarrier-System, umfassend:
- einen inneren Kern umfassend ein Nanopartikel und/oder zumindest eine pharmazeutische Verbindung;
- einen ersten Liganden, der an die Oberfläche des inneren Kerns gebunden ist;
- einen zweiten Liganden, der an den ersten Liganden gebunden ist, wobei der zweite Ligand weiterhin kovalent an Poly(ethylenglykol) oder einem Derivat davon gebunden ist,
**dadurch gekennzeichnet, dass** die Bindung des zweiten Liganden an den ersten Liganden nichtkovalent und reversibel ist, wobei der erste Ligand und der zweite Ligand aus den folgenden Kombinationen von ersten/zweiten Liganden ausgewählt werden:
| **Erster Ligand** | **Zweiter Ligand** |
|---|---|
| Fluorescein, FITC | scFv Fragment FITC-E2 |
| Fluorescein, FITC | scFv Fragment FITC-E2 gemäß SEQ ID NO: 1 oder 19 oder eine Sequenz, die mindestens 80% Sequenzidentität zur Sequenz gemäß SEQ ID NO: 1 oder 19 aufweist; |
| Biotin | anti-Biotin scFv Fragment |

2. Reversibel PEGyliertes Nanocarrier-System gemäß Anspruch 1, wobei das Nanocarrier-System weiterhin mindestens eine Lipid-Doppelschicht auf der Oberfläche des inneren Kerns umfasst, wobei der erste Ligand dann an die Oberfläche mindestens einer Lipid-Doppelschicht gebunden ist.

3. Reversibel PEGyliertes Nanocarrier-System gemäß Anspruch 2, wobei das Nanocarrier-System eine mono-oder eine multilamellare Lipid-Doppelschicht auf der Oberfläche des inneren Kerns aufweist, wobei der erste Ligand an die Oberfläche dieser mindestens einen Lipid-Doppelschicht gebunden ist.

4. Reversibel PEGyliertes Nanocarrier-System gemäß einem der Ansprüche 1-3, wobei der zweite Ligand kovalent an Poly(ethylenglykol) oder einem Derivat davon gebunden ist.

5. Reversibel PEGyliertes Nanocarrier-System gemäß einem der Ansprüche 1-4, wobei das Molekulargewicht des Poly(ethylenglykol)s oder einem Derivat davon im Bereich zwischen 500 und 10.000 gmol⁻¹ liegt, bevorzugt zwischen 1.000 und 8.000 gmol⁻¹, stärker bevorzugt zwischen 1.000 und 5.000 gmol⁻¹ liegt.

6. Reversibel PEGyliertes Nanocarrier-System gemäß einem der Ansprüche 1-5, wobei das Nanocarrier-System eine Größe von etwa 10-1.500 nm, bevorzugt 10-1.200 nm, gleicherweise bevorzugt 10-1.000 nm, 10-800 nm, 10-600 nm oder 10-400 nm, noch stärker bevorzugt 20-300 nm, 50-250 nm oder 50-200 nm aufweist.

7. Reversibel PEGyliertes Nanocarrier-System gemäß einem der Ansprüche 1-6, wobei die mindestens eine pharmazeutische Verbindung ein tumoricidales Agens ist ausgewählt aus der Gruppe umfassend 2,4,6-Triethylenmelamin, 6-Mercaptopurin, ACNU, Actinomycin D, Adenosindialdehyd, Adriamycin, AE-941, AG3340, Andaminopterin, anti-VEGF Antikörper, Bay 12-9556, BCNU, Bleomycin, BM S-275291, Busulfan, CA I, Camptothecin, Carboplatin, CCNU, Chlorambucil, Cisplatin, COL-3, Cyclophosphamid, Cytarabin, Dacarbazin, Dasatinib, Daunorubicin, Doxorubicin, EMD 121974, Endostatin, Epirubicin, Erlotinib, Etoposid, Floxuridin, Fluorouracil, Ftorafur, Furtulon, Gefitinib, Guanazol, Harringtonin, HXM, Hydroxycamptothecin, Hydroxyurea, Ifosfamid, IM8624, Imatinib, Indirubin, Inosinedialdehyd, Interferon [alpha], Interleukin 12, Irinotecan, lapatinib, liposomale p53 DNA, Marimastat, Methotrexate, Methyl CCNU, Mithramycin, Mitomycin A, Mitomycin B, Mitomycin C, Mitoxantron, Navelbin, Neovastat, Nilotinib, Nitrogen-Mustard, N-methyl-N-Nitrosourea, Olivomycin, Oxaliplatin, Paclitaxel, Pingyangmycin, Pirarubicin, PKN3siRNA, Procarbazin, Sorafenib, Squalamin, Streptozotocin, SU5416, SU6668, Sunitinib, Suramin, Taxoter, Tegadifur, Tegafur-Uracil, Thalidomide, Thiotepa, TNP-470, Vinblastin, Vincristin, Vindesin, Vinorelbin, und/oder Vitaxin, oder eine Kombination davon, und/oder die mindestens eine pharmazeutische Verbindung wird ausgewählt aus einem Gen-therapeutischen Mittel, umfassend DNA, pDNA, RNA, siRNA, miRNA, oder eine Kombination davon.

8. Reversibel PEGyliertes Nanocarrier-System gemäß einem der Ansprüche 1-7, wobei die mindestens eine pharmazeutische Verbindung DNA, RNA, pDNA oder siRNA umfasst oder daraus ausgewählt wird, oder wobei der Nanopartikel solch eine pharmazeutische Verbindung umfasst.

9. Reversibel PEGyliertes Nanocarrier-System gemäß einem der Ansprüche 1-8, wobei der Nanopartikel Dextran oder ein Polymer umfasst, ausgewählt aus PEG, PLA, PLGA, oder eine inerte Verbindung, oder ein Vesikel sein kann.

10. Reversibel PEGyliertes Nanocarrier-System gemäß einem der Ansprüche 1-9, wobei das Nanocarrier-System einen dritten Liganden umfasst, der an das Nanocarrier-System gebunden ist, um zielgerichtet das reversibel PEGylierte Nanocarrier-System zu einer bevorzugten Target-Region zu führen.

11. Reversibel PEGyliertes Nanocarrier-System gemäß einem der Ansprüche 1-10, wobei der genannte dritte Ligand ausgewählt wird aus anti-HER2, anti-CD9, Nukleosom-spezifischem 2C5 mAb, Transferrin, Interleukin 13 (IL-13), Octreotid, LHRH-basiertem Peptid, Arg-Gly-Asp (RGD), Folat, Estron, Anisamid, Mannose, und/oder Laktose.

12. Pharmazeutische Zusammensetzung, umfassend das reversibel PEGylierte Nanocarrier-System gemäß einem der Ansprüche 1-11 und optional einen pharmazeutisch geeigneten Träger und/oder Vehikel.

13. Reversibel PEGyliertes Nanocarrier-System gemäß einem der Ansprüche 1-11 zur Verwendung als Medikament.

14. Reversibel PEGyliertes Nanocarrier-System gemäß einem der Ansprüche 1-11, oder eine pharmazeutische Zusammensetzung gemäß Anspruch 12 zur Verwendung in der Krebstherapie, Gentherapie, Schmerztherapie oder zur Behandlung von entzündlichen Erkrankungen.

## Revendications

1. Système de nanosupport PEGylé réversiblement, comprenant :
- un cœur interne comprenant une nanoparticule et/ou au moins un composé pharmaceutique ;
- un premier ligand lié à la surface dudit cœur interne ;
- un deuxième ligand lié au premier ligand, le deuxième ligand étant en outre lié de façon covalente à du poly(éthylèneglycol) ou un dérivé de celui-ci,
**caractérisé en ce que** la liaison du deuxième ligand au premier ligand est non covalente et est réversible, dans lequel le premier ligand et le deuxième ligands sont choisis parmi les combinaisons de premier ligand/deuxième ligand suivantes :
| Premier ligand | Deuxième ligand |
|---|---|
| Fluorescéine, FITC | fragment scFv FITC-E2 |
| Fluorescéine, FITC | fragment scFv FITC-E2 selon SEQ ID NO: 1 ou 19 ou une séquence présentant au moins 80% d'identité avec la séquence de SEQ ID NO: 1 ou 19 |
| Biotine | Fragment scFv anti-biotine |

2. Système de nanosupport PEGylé réversiblement selon la revendication 1, lequel système de nanosupport comprend en outre au moins une bicouche lipidique sur la surface du cœur interne, dans lequel le premier ligand est alors lié à la surface d'au moins une bicouche lipidique.

3. Système de nanosupport PEGylé réversiblement selon la revendication 2, lequel système de nanosupport a une bicouche lipidique mono- ou multilamellaire sur la surface du cœur interne, dans lequel le premier ligand est lié à la surface de ladite au moins une bicouche lipidique.

4. Système de nanosupport PEGylé réversiblement selon l'une quelconque des revendications 1 à 3, dans lequel le deuxième ligand est lié de façon covalente à du poly(éthylèneglycol) ou un dérivé de celui-ci.

5. Système de nanosupport PEGylé réversiblement selon l'une quelconque des revendications 1 à 4, dans lequel le poids moléculaire du poly(éthylèneglycol) ou d'un dérivé de celui-ci est dans la gamme entre 500 et 10000 g/mole, de préférence entre 1000 et 8 000 g/mole, de préférence encore entre 1 000 et 5 000 g/mole.

6. Système de nanosupport PEGylé réversiblement selon l'une quelconque des revendications 1 à 5, lequel système de nanosupport a une dimension d'environ 10 à 1 500 nm, de préférence 10 à 1 200 nm, de préférence encore 10 à 1 000 nm, 10 à 800 nm, 10 à 600 nm ou 10 à 400 nm ou de préférence encore 20 à 300 nm, 50 à 250 nm ou 50 à 200 nm.

7. Système de nanosupport PEGylé réversiblement selon l'une quelconque des revendications 1 à 6, dans lequel ledit au moins un composé pharmaceutique est un agent tumoricide choisi dans le groupe constitué par un composé de 2,4,6-triéthylène mélamine, 6-mercaptopurine, ACNU, actinomycine D, adénosinediialde-hgde, adriamycine, AE-941, AG3340, andaminoptérine, anticorps anti-VEGF, Bay 12-9556, BCNU, bléomycine, BM S-275291, busulfan, CA I, camptothécine, carboplatine, CCNU, Chlorambucil, cisplatine, COL-3, cyclophosphamide, cytarabine, dacarbazine, dasatinib, daunorubicine, doxorubicine, EMD 121974, endostatine, épirubicine, erlotinib, étoposide, floxuridine, fluorouracile, ftorafur, furtulon, géfitinib, guanazole, harringtonine, HXM, hydroxy camptothécine, hydroxyurée, ifosfamide, IM8624, imatinib, indirubine, inosine dialdéhyde, interféron [alpha], interleukine 12, irinotécan, lapatinib, ADN p53 liposomique, marimastat, méthotrexate, méthyl CCNU, mithramycine, mitomycine A, mitomycine B, mitomycine C, mitoxantrone, navelbine, néovastat, nilotinib, moutarde azotée, N-méthyl-N-nitrosourée, olivomycine, oxaliplatine, paclitaxel, pingyangmycine, pirarubicine, RNAsi de PKN3, procarbazine, sorafénib, squalamine, streptozotocine, SU5416, SU6668, sunitinib, suramine, taxotère, tégadifur, tégafur-uracil, thalidomide, thiotépa, TNP-470, vinblastine, vincristine, vindésine, vinorelbine et/ou vitaxine, ou une combinaison de ceux-ci, et/ou ledit au moins un composé pharmaceutique est un agent thérapeutique génique.

8. Système de nanosupport PEGylé réversiblement selon l'une quelconque des revendications 1 à 7, dans lequel ledit au moins un composé pharmaceutique comprend ou est choisi parmi un ADN, un ARN, un ADNp ou un ARNsi, ou dans lequel la nanoparticule comprend un tel composé pharmaceutique.

9. Système de nanosupport PEGylé réversiblement selon l'une quelconque des revendications 1 à 8, dans lequel la nanoparticule comprend du dextrane ou un polymère choisi parmi PEG, PLA, PLGA ou un composé inerte, ou peut être une vésicule.

10. Système de nanosupport PEGylé réversiblement selon l'une quelconque des revendications 1 à 9, lequel système de nanosupport comprend un troisième ligand attaché au système de nanosupport pour cibler spécifiquement le système de nanosupport PEGylé réversiblement à une région cible souhaitée.

11. Système de nanosupport PEGylé réversiblement selon la revendication 10, dans lequel ledit troisième ligand est choisi parmi un anti-HER2, anti-CD9, anticorps monoclonal 2C5 spécifique à un nucléosome, transferrine, interleukine 13 (IL-13), Octréotide, peptide dérivé de LHRH, Arg-Gly-Asp (RGD), folate, estrone, anisamide, mannose et/ou lactose.

12. Composition pharmaceutique comprenant le système de nanosupport PEGylé réversiblement selon l'une quelconque des revendications 1 à 11 et éventuellement un support et/ou véhicule pharmaceutiquement acceptable.

13. Système de nanosupport PEGylé réversiblement selon l'une quelconque des revendications 1 à 11, pour utilisation en tant que médicament.

14. Système de nanosupport PEGylé réversiblement selon l'une quelconque des revendications 1 à 11 ou composition pharmaceutique selon la revendication 12, pour utilisation en thérapie anticancéreuse, thérapie génique, thérapie de douleur ou traitement de maladies inflammatoires
